# EUROPEAN PATENT SPECIFICATION

(11) **EP 1 466 011 B1**
(45) Date of publication and mention of the grant of the patent: **11.10.2006**
(21) Application number: 02786262.2
(22) Date of filing: 19.12.2002
(51) Int. Cl.: C12Q 1/68

(54) **NEW PRIMERS FOR THE DETECTION AND IDENTIFICATION OF BACTERIAL INDICATOR GROUPS AND VIRULENCE FACTORS**
NEUE PRIMER ZUM NACHWEIS UND ZUR IDENTIFIZIERUNG BAKTERIELLER INDIKATORGRUPPEN UND VIRULENZFAKTOREN
NOUVELLES AMORCES POUR LA DETECTION ET L'IDENTIFICATION DE GROUPES D'INDICATEURS BACTERIENS

(30) Priority: 19.12.2001 NO 20016251; 19.12.2001 US 340872 P
(43) Date of publication of application: 13.10.2004
(73) Proprietor: Angles D'Auriac, Marc B., 0663 Oslo (NO)
(72) Inventor: ANGL S D'AURIAC, Marc, B., N-0663 Oslo (NO); SIREV G, Reidun, N-0371 Oslo (NO)
(74) Representative: Baldock, Sharon Claire
(86) International application number: PCT/NO2002/000490
(87) International publication number: WO 2003/052143

(56) References cited:
- WO-A-98/48046
- WO-A-99/41614
- CA-A- 2 286 512
- OSEK J ET AL: "Development of a PCR-based method for specific identification of genotypic markers of shiga toxin-producing ESCHERICHIA COLI strains." JOURNAL OF VETERINARY MEDICAL SERIES B, vol. 48, no. 10, 2001, pages 771-778, XP002902921 2001 Blackwell Wissenschafts-Verlag, Berlin ISSN: 0931-1793
- BEILEI GE ET AL: "A PCR-ELISA for detecting shiga toxin-producing ESCHERICHIA COLI ." MICROBES AND INFECTION, vol. 4, 2002, pages 285-290, XP002902922 Elsevier SAS ISSN: S1286-4579 -& DATABASE REGISTRY [Online] XP002902932
- MANSOUR SAMADPOUR ET AL: "Development and evaluation of oligonucleotide DNA probes for detecting and genotyping of shiga-like toxin producing ESCHERICHIA COLI." JOURNAL OF FOOD PROTECTION, vol. 57, no. 5, May 1994 (1994-05), pages 399-402, XP002902923 -& DATABASE REGISTRY [Online] XP002902933
- PATON A W ET AL: "Detection and characterization of shiga toxigenic ESCHERICHIA COLI by using mutiplex PCR assays for stx1, stx2, eaeA, enterohemorrhagic E. coli hlyA, rbfo111, and rbfo157." JOURNAL OF CLINICAL MICROBIOLOGY, WASHINGTON, DC, US, vol. 36, no. 2, February 1998 (1998-02), pages 598-602, XP000908789 ISSN: 0095-1137 cited in the application
- FAGAN P K ET AL: "Detection of shiga-like toxin (stx1 and stx2), intimin (eaeA),and enterohemorrhagic ESCHERICHIA COLI (EHEC) hemolysin (EHEC hlyA) genes in animal feces by multiplex PCR." APPLIED AND ENVIRONMENTAL MICROBIOLOGY, vol. 65, no. 2, February 1999 (1999-02), pages 868-872, XP002902924 ISSN: 0099-2240 cited in the application
- BASTIAN S N ET AL: "COMPARISON OF 14 PCR SYSTEMS FOR THE DETECTION AND SUBTYPING OF STX GENES IN SHIGA-TOXIN-PRODUCING ESCHERICHIA COLI" RESEARCH IN MICROBIOLOGY, AMSTERDAM, NL, vol. 149, no. 7, July 1998 (1998-07), pages 457-472, XP001053308
- BATCHELOR M ET AL : "Development of a universal intimin antiserum and PCR primers." JOURNAL OF CLINICAL MICROBIOLOGY, vol. 37, no. 12, December 1999 (1999-12), pages 3822-3827, XP002902925 ISSN: 0095-1137
- MC DANIELS A E ET AL: "Confirmational identification of ESCHERICHIA COLI, a comparison of genotypic anf phenotypic assays for glutamate decarboxylase and beta-D-glucuronidase." APPLIED AND ENVIRONMENTAL MICROBIOLOGY, vol. 62, no. 9, September 1996 (1996-09), pages 3350-3354, XP002902926 ISSN: 0099-2240 cited in the application
- GRANT M A ET AL : "Glutamate decarboxylase genes as a prescreening marker for detection of pathogenic ESCHERICHIA COLI groups." APPLIED AND ENVIRONMENTAL MICROBIOLOGY, vol. 67, no. 7, July 2001 (2001-07), pages 3110-3114, XP002902927 ISSN: 0099-2240 cited in the application
- AN F Y ET AL: "Identification and characterization of determinant (EEP) on the ENTEROCOCCUS FAECALIS chromosome that is involved in production of the peptide sex pheromone cAD1." JOURNAL OF BACTERIOLOGY, vol. 181, no. 19, October 1999 (1999-10), pages 5915-5921, XP002902928 ISSN: 0021-9193 cited in the application
- KOBAYASHI N ET AL: "Distribution of aminoglycoside resistance genes in recent clinical isolates of ENTEROCOCCUS FAECALIS, ENTEROCOCCUS FAECIUM and ENTEROCOCCUS AVIUM." EPIDEMIOL. INTERF., vol. 126, 2001, pages 197-204, XP002902929
- COSTA Y ET AL : "Characterization of the chromosomal AAC(6')-Ii gene specific for ENTEROCOCCUS FAECIUM." ANTIMICROBIAL AGENTS AND CHEMOTHERAPY, vol. 37, no. 9, September 1993 (1993-09), pages 1896-1903, XP002902930 ISSN: 0066-4804 cited in the application
- BAYARDELLE P ET AL : "Development of oligonucleotide primers for the specific PCR-based detection of the most frequent ENTEROBACTERIACEAE species DNA using WEC gene templates." CANADIAN JOURNAL OF MICROBIOLOGY, vol. 48, no. 2, 2002, pages 113-122, XP002902931

### SEQUENCE LISTINGS

<110> Marc B. Angles d'Auriac
<120> New primers for the detection and identification of bacterial indicator groups and virulence factors
<130> 114792/KHS
<160> 30
<210> 1
   <211> 24
   <212> DNA
   <213> Virulence factor genes found in species of the Enterobacteriaceae family
<400> 1
   trttgarcra aataatttat atgtg
<210>2
   <211> 21
   <212> DNA
   <213> Virulence factor genes found in species of the Enterobacteriaceae family
<400> 2
   mtgatgatgr caattcagta t
<210> 3
   <211> 22
   <212> DNA
   <213> Virulence factor genes found in species of the Enterobacteriaceae family
<400> 3
   cmtgatgatg rcaattcagt at
<210> 4
   <211> 24
   <212> DNA
   <213> Virulence factor genes found in species of the Enterobacteriaceae family
<400> 4
   aatggaacgg aataacttat atgt
<210> 5
   <211> 21
   <212> DNA
   <213> Virulence factor genes found in species of the Enterobacteriaceae family
<400> 5
   ggttgagtgg caattaagga t
   <210> 6
   <211> 21
   <212> DNA
   <213> Virulence factor genes found in species of the Enterobacteriaceae family
<400> 6
   attatggaac ggcagaggtt a
<210> 7
   <211> 21
   <212> DNA
   <213> Virulence factor genes found in species of the Enterobacteriaceae family
<400> 7
   tgaagacgtt atagcccaac a
<210> 8
   <211> 21
   <212> DNA
   <213> Virulence factor genes found in species of the Enterobacteriaceae family
<400> 8
   ggcgctcatc atagtctttc t
<210> 9
   <211> 18
   <212> DNA
   <213> Virulence factor genes found in species of the Enterobacteriaceae family
<400> 9
   acccggcaca agcataag
<210> 10
   <211> 21
   <212> DNA
   <213> Virulence factor genes found in species of the Enterobacteriaceae family
<400> 10
   cgtaaagcgr gagtcaatrt a
<210> 11
   <211> 21
   <212> DNA
   <213> Virulence factor genes found in species of the Enterobacteriaceae family
<400> 11
   ggcgctcatc atagtctttc t
<210> 12
   <211> 21
   <212> DNA
   <213> Enterobacteriaceae family
<400> 12
   tggatatggt ggcgattatg t
<210> 13
   <211> 18
   <212> DNA
   <213> Enterobacteriaceae family
<400> 13
   tccaggcmcg cttaatgc
<210> 14
   <211> 21
   <212> DNA
   <213> Enterobacteriaceae family
<400> 14
   cyttccaggc mcgcttaatg c
<210> 15
   <211> 18
   <212> DNA
   <213> Enterobacteriaceae family
<400> 15
   ttcccgycag gcrtttgt
<210> 16
   <211> 21
   <212> DNA
   <213> Enterobacteriaceae family
<400> 16
   cmggyawtgg ttgtgtcatc r
<210> 17
   <211> 20
   <212> DNA
   <213> Enterobacteriaceae family
<400> 17
   gggttrtccw gcgtctcrtt
<210> 18
   <211> 21
   <212> DNA
   <213> Enterobacteriaceae family
<400> 18
   tattctgccr kyacgccway k
<210> 19
   <211> 21
   <212> DNA
   <213> *Escherichia coli*
<400> 19
   aaagaagaat atccgcaatc c
<210> 20
   <211> 17
   <212> DNA
   *<213> Escherichia coli*
<400> 20
   gccatttcat cgccatc
<210> 21
   <211> 19
   <212> DNA
   *<213> Escherichia coli*
<400> 21
   ccacaaccgc tgcacgatg
<210> 22
   <211> 21
   <212> DNA
   *<213> Escherichia coli*
<400> 22
   caggcggaag tcccagacga t
<210> 23
   <211> 21
   <212> DNA
   *<213> Enterococcus faecalis*
<400> 23
   aatgccgtgg gtaatgtggt t
<210> 24
   <211> 20
   <212> DNA
   *<213> Enterococcus faecalis*
<400> 24
   ggcttttcgg ggttcttctg
<210> 25
   <211> 21
   <212> DNA
   *<213> Enterococcus faecalis*
<400> 25
   ttgagttaaa tgccgtgggt a
<210> 26
   <211> 16
   <212> DNA
   *<213> Enterococcus faecalis*
<400> 26
   catgggtccc gcaaag
<210> 27
   <211> 21
   <212> DNA
   *<213> Enterococcus faecium*
<400> 27
   gggggaagac gtatgataat c
<210> 28
   <211> 21
   <212> DNA
   *<213> Enterococcus faecium*
<400> 28
   tcgggagctt tctacaacta a
<210> 29
   <211> 20
   <212> DNA
   <213> *E. faecium*
<400> 29
   ggcgtattta acttagtcgt
<210> 30
   <211> 20
   <212> DNA
   <213> *Enterococcus faecium*
<400> 30
   tttgcgtctt ctcgtaattt
<210> 31
   <211> 20
   <212> DNA
   <213> Virulence factor genes found in species of the Enterobacteriaceae family
<400> 31
   gtacaacact kgatgatctc
<210> 32
   <211> 17
   <212> DNA
   <213> Virulence factor genes found in species of the Enterobacteriaceae family
<400> 32
   tgacracgga cagcagt

## Description

The present invention relates to new primers for the detection of any of three important bacterial indicator groups used in food microbiology and two virulence factors, which are associated with the aetiology of several types of watery and bloody human diarrhoea. Furthermore, the present invention also relates to use of the primers in a method which enables this detection, as well as the detection of new emerging pathogenic bacteria.

There is a great need today for methods to secure safe sanitary (i.e. the absence of harmful bacteria) evaluation of goods for human consumption, in particular water, as well as methods for the detection of pathogen bacteria useful for e.g. clinical diagnostic. Three important target bacterial indicator groups (made of various species) currently used in food microbiology are the Enterobacteriaceae (family), *Escherichia coli* (*E. coli*; a species belonging to the Enterobacteriaceae family) and the fecal enterococci (most species of the *Enterococcus* genus).

The Enterobacteriaceae is a coherent well-defined taxonomic unit, which is relevant both to clinical diagnostic and to food and water routine microbiological analysis, as it includes important human pathogens and the total coliform group. Traditional microbiological methods used for the identification of this family rely on biochemical properties of isolated re-grown bacterial colonies. Only few faster alternative methods have been developed so far, and they are based on the identification of a trait or marker specific to the taxon. Sequences of 16S rRNA genes have been widely used for phylogenetic and taxonomic analysis as well as for diagnostic applications, i.e. for the detection of Enterobacteriaceae members (Mittelman *et al.* 1997).

The Enterobacterial Common Antigen (ECA) was first described in 1963 by Kunin et al. (Kunin 1963) and defined as a cross-reactive antigen that is detectable in all genera of Enterobacteriaceae by indirect hemagglutination and by other methods using antiserum to *E. coli.* It was later found to be strictly family specific with diagnostic and prophylactic potential. The only known noticeable reported exceptions are the Enterobacteriacea ECA-negative *Erwinia chrysanthemi* and the non-Enterobacteriacea ECA-positive *Plesiomonas shigelloides,* both of which have disputed taxonomic positions (see review (Kuhn *et al.* 1988)). The ECA is a glycophospholipid built up by an aminosugar heteropolymer linked to an L-glycerophosphatidyl residue. This surface antigen remained undetected for a long time due to its non-immunogenicity in most Enterobacteriaceae despite its general ability to act as an epitope (hapten). The genes implicated in the synthesis of ECA, *rfe* and *rff,* are clustered around 85 min on the *E. coli* genome (Ohta *et al.* 1991).

Immunology-based diagnostic tests have been developed to detect the presence of ECA for clinical applications (Malkamaki 1981) and later to monitor drinking water microbiological quality by detecting bacteria belonging to the Enterobacteriaceae family (Hubner *et al.* 1992). Such tests rely on the expression of the character being screened, which might be absent or poorly expressed in mutants, although most of the coding material may still remain intact. In this connection DNA-based techniques, i.e. PCR, have been successfully used to decrease the amount of false negatives in diagnostic applications, i.e. beta-glucuronidase enzyme and its coding sequence used for the detection and identification of *E. coli* (Feng *et al.* 1991). However, in order to be efficient and practical, it is important that the PCR methods that are used are robust, i.e. that they provide a strong and easily reproducible amplification, with no generation of additional product. Furthermore, the use of multiple primer sets in the same PCR reaction (i.e. multiplex PCR; two primer pairs means a duplex PCR, three primer pairs a triplex PCR etc.) is also preferable to the use of a separate PCR protocol for each of the primer sets (i.e. simplex PCR) when multiple targets are searched. This allows saving time and reagents, and thus lowering the cost of the analysis.

When applying PCR it is possible to use so called universal primers. Universal primers have the purpose of working for all variants of a given gene or DNA target. Typically the primers will be chosen in the most conserved areas of the gene, ideally identical in all variants. When no conserved identical portion can be used, two strategies can be used to accommodate the ambiguous nucleotide positions: silent mismatch and degenerate primers. In the first case, the primers are designed so that the variable nucleotide positions are placed in the primers to allow amplification to proceed although there is one or more mismatch. Typically, these ambiguous positions will be placed at the 5' end of the primers. In the case of degenerate primers, all the ambiguous positions are accounted for, and a mix of all possible combination of the variable positions is used. This has the inconvenience of diluting the one full match primer set. However, the advantage is that it will be more efficient for highly variable genes and have more chances of functioning on new unknown variants of the target.

*E. coli* is a member of the Enterobacteriaceae and the main species of the thermotrophic coliform group, also called the faecal coliform group. In the UK, the Drinking Water Inspectorate advised the committee responsible for revising Report 71 (Public Health Laboratory Services 1994) that for regulatory purposes, confirmed *E. coli* can be regarded as faecal coliforms. Furthermore, as *E. coli* is viewed as the only true faecal coliform and constitutes up to 99% of all faecal coliform isolates, its detection has been recommended for the evaluation of water microbiological quality. Traditional microbiological methods have relied on the expression of specific biochemical properties such as fermenting lactose or manitol at 44°C with the production of acid and usually gas within 24 hours, and the production of indole from tryptophane. The expression by most *E. coli* strains of the β-glucuronidase has also been exploited. As previously mentioned, such tests rely on the expression of the specific characteristic which may lack or be delayed among certain strains although the genes might still be present. Hence, DNA-based methods, i.e. PCR, not only will reduce the analysis time, but also reduce the amount of false negatives when a reliable specific target gene or signature sequence within the chosen gene is used. Various PCR methods have been developed for detecting *E. coli* based on the detection of *uidA* (*β-*glucuronidase) and *lamB* (maltose high-affinity uptake system) (Bej *et al.* 1991b), or *gadAB* (glutamate decarboxylase) (McDaniels *et al.* 1996) which was also successfully used for pathogenic *E. coli* (Grant *et al.* 2001).

*Enterococcus* is catalase-negative Gram-positive facultative anaerobic bacteria, and the two major species of interest to humans are *Enterococcus faecalis* (*E. faecalis*) and *Enterococcus faecium* (*E. faecium*). They are very common intestine commensal but are also responsible for nosocomial bacteremia, surgical wound infection, endocarditis and urinary tract infection. Most infections are caused by *E. faecalis,* which has virulence genes, whereas *E. faecium* has not but is more often resistant to glycopeptides (vancomycin and teicoplanin). Because of the lack of biochemical diversity between enterococcal species, reliable identification using traditional microbiological tests has proven to be difficult. This has become a problem for infection control purposes, as accurate species identification is required for the appropriate antibiotic treatment of enterococci infections. For example, *E. faecalis* usually are susceptible to ampicillin whereas vancomycin-resistant *E. faecium* also express high levels of resistance to β-lactams.

In addition of being clinically relevant species, *E. faecalis* and *E. faecium* are the two main species of the faecal enterococci indicator group used for the microbiological assessment of food and water. PCR methods have been developed to detect other enterococcal species using for example the *vanC-1, vanC-2* and *vanC-3* genes of *E. gallinarum, E. casseliflavus* and *E. flavescens* respectively, coding for their specific intrinsic low resistance to glycopeptides (Dutka-Malen *et al.* 1995). Other nucleotide based methods have been using housekeeping genes such as 23S rRNA gene (Betzl *et al.* 1990), super oxide dismutase gene (Bergeron *et al.* 1999) or randomly selected specific DNA sections (Cheng *et al.* 1997) for the specific detection of *E. faecalis* and *E. faecium.* Finally, *tuf* coding for the elongation factor EF-Tu has been used for the identification of the *Enterococcus* genus (Ke *et al.* 1999). However, none of these methods managed to simultaneously detect *E. faecalis* and *E. faecium* nor did they use a specific gene for the detection. House keeping genes were used which reduces the chances of developing a robust PCR or to further develop a multiplex method.

In addition to the detection of bacterial indicator groups, in which different species or sub-species (may) share similar genes for the coding of specific virulence factors, methods to detect the virulence factor themselves are important. Two virulence factors found in e.g. enteropathogenic *E. coli* (EPEC) and enterohemorrhagic *E. coli* (EHEC) are Shiga toxin (Stx) encoded by the gene *stx* and Intimin encoded by the gene *eae.* These two virulence factors are known as the two main virulence factors associated with the onset of human diarrhoea symptoms by these bacterial pathogens (i.e. EPEC and EHEC).

The Shiga toxin class, as indicated by its name, was first discovered in *Shigella dysenteriae* type 1 bacteria. A similar toxin was later discovered in *E. coli,* characterized as cytotoxic to vero cells and named Vero toxin (VT). The group of *E. coli* producing VT was accordingly named VTEC. The VT was later shown to be related to Shiga toxin, which prompted some authors to rename it Shiga like toxin (SLT), and the term SLTEC was used to describe the bacterial group (i.e. *E. coli*) producing it. As it became more evident that all Shiga toxins are related, a new genetic nomenclature was proposed and widely accepted (and is the one we use in the present study), and consequently this group of *E. coli* is now referred to as Shiga toxin producing *E. coli* (STEC).

Many variants of the *stx* gene have been described and new ones are still characterized. They have been classified in 2 main groups according to their sequence similarity. The first, *stx*₁*,* is found in STEC and are almost identical to the shiga toxin genes of *S. dysenteriae* type 1, *stx.* The second group, *stx*₂ and variants, is the most divergent and comprises sub-groups which appear to be found in host-adapted strains and other species than *E. coli,* and also encode for the most potent shiga toxin for humans. Both *stx*₂ and *stx*_{2c} are mainly hosted by STEC associated with the aetiology of severe human diarrhoea, whereas *stx*_{2d} has been isolated in STEC of both human and cattle origin. Finally, *stx*₂ₑ are found in porcine *E. coli* while *stx*_{2f} are found in *E. coli* hosted by birds. Although Stx2e and Stx2f toxins seem to be adapted to their respective hosts, they both have been associated with human disease. Combination of different *stx* variants can be found in a same bacteria as illustrated by the case of a patient with three different STEC serotypes, each of which was hosting Stx1, Stx2 and Stx2c. Cattle are considered to be the main reservoir of STEC with 50 to 95% of the animals found to be host, although many other domestic animals were also found to host STECs. It was also shown that bacteria carrying *stx* genes were isolated from marine waters and are commonly found in rivers. Although not all Stx-producing bacteria can have phage induced, all *stx* genes are considered to be phage borne, including for *S. dysenteriae* serotype 1. In this connection, Shiga toxin-converting bacteriophages are commonly isolated in sewage and were shown to play an important role in the emergence of new STEC variants. These findings illustrates how ubiquitous Shiga toxins are in our environment spanning from land to sea and air, with the intrinsic potential of horizontally spreading to new bacterial hosts.

The Shiga toxin is an A-B toxin type formed of the association of 5 B subunits structured in a ring-alike shape, and one A subunit on top of the ring. The ring is responsible for the recognition and attachment to the eukaryotic Gb3 globotriaosylceramide cell receptor of the toxin whereas the A subunit is the active toxic component that inhibits protein synthesis by removing an adenine from the 28 S rRNA. The two subunits are encoded by two genes organized in an operon in which the B subunit is more transcribed than A, enabling the final molecular ratio of 1/5 for the whole toxin. The *stxA* gene varies in length from 948 bp for *stxA*₁ to around 960 bp for *stxA*₂, and the "theoretical" maximum length after alignment of all variants is 967 bp and is used as the reference template for numbering the primers as shown in Figure 9. The B subunit is 267 bp in length. As more *stxA* sequences were described than *stxB,* and as *stxA* is longer, we chose the latter (i.e. *stxA*) for the development of universal primers to detect the presence of *stx.*

Although the first and main STEC serotype associated with the onset of human disease is O157:H7, over a 100 serotypes have been recognized and thus, the importance of developing methods for detecting them has been emphasized (World Health Organization 1998). The STEC serotype associated with the development of human haemolytic uraemic syndrome (HUS) might vary from a country to another as shown in a recent Australian survey in which non of the 98 HUS cases identified over 4 years were associated to O157:H7. Similarly, in another Australian study, no O157:H7 were isolated among the 23 STEC isolated from bovine faecal samples. Non-O157 STEC were possibly previously underestimated because of the use of diagnostic methods targeting typical phenotypic characteristics of the O157:H7 serotype such as delayed sorbitol fermentation and lack of glucuronidase activity rather than toxin detection. These tests were developed to enable mass screening by routine laboratory but will obviously miss many STEC including atypical O157 isolates. The same critic can be made for serological diagnostic tests, which are specific to the serotype, i.e. O157 detection methods. Other immunological diagnostic methods targeting Shiga toxins have been developed but rely on toxin expression and lack the analytical flexibility DNA-based methods have. To circumvent the unreliability of phenotypic expression, it is clear that a DNA-based method able to detect all variants of the gene encoding Shiga toxin is needed when evaluating human health risk of environmental samples or when identifying aetiological agents of human gastroenteritis. Although various universal primer pairs for the detection of *stx* have been described in the literature (see Figure 1 and Table 4), few are able to detect all variants or have been used in a multiplex assay.

The *eae* gene (E. coli attaching and effacing) encodes Intimin of pathogenic *E. coli* producing the typical A/E (attaching-and-effacing) histopathology in infected patients. Five different types have been described: α, β, γ, δ & ε. The open reading frame varies in length from 2820 bp for intimin α and β to 2847 bp for intimin ε. Intimin is a protein involved in the intimate adherence of the bacterium to the epithelial cell membrane of the host's gut. In an experiment with human volunteers, intimin was proven to be necessary for the full development of diarrhoea caused by EPEC. The *eae* gene is found in the so-called locus of enterocyte effacement (LEE) pathogenecity island of both EPEC and EHEC. The location of LEE on the chromosome rather than on a plasmid, which is often the case for several other virulence factors, is beneficial in terms of stability of that DNA segment. Plasmid loss during subculturing has been reported and demonstrates that pathogenic plasmid borne molecular markers might be unreliable.

Several patents disclose methods to detect harmful bacteria. US 6207818, US 6060252, US 6054269 and US 5298392 all describe the amplification and detection of such harmful bacteria, however, the methods either detect different indicator markers/groups (i.e. bacteria) and/or different virulence markers/factors (i.e. gene(s)) compared to what is disclosed in the present application. Furthermore, the methods used in these patents do not combine the Enterobacteriaceae indicator group with virulence markers.

US 6218110, US 6165724 and US 5795717 use oligonucleotides in PCR protocols to detect STEC bacteria. However, as opposed to what is disclosed in the present application, they use 2 or more sets of primers for the detection of *stx* and all variants.

US 5652102 use oligonucleotides in a multiplex PCR protocol to detect STEC bacteria, however, as opposed to what is disclosed in the present application, the primer pairs are claimed to be specific to the E. coli O157 serogroup and no indicator group is associated to the method.

US 5994066, US 5786147 and US 5693469 use a "housekeeping gene" like rRNA and/or probe technology (as opposed to PCR) to detect various bacteria, and is thus more limited and/or cumbersome than the method disclosed in the present application.

Two articles by Fratamico et al. (Fratamico *et al.* 1993) disclose the use of multiplex PCR protocols using universal primers for *stx;* however, these primers are not able to detect the *eae* gene and variants. Furthermore, these protocols use mismatched universal primer pairs developed by Karch et al. (Karch and Meyer 1989), which was later reported not to detect all variants originally claimed.

Osek et al. (Osek 2001) have reported the development of a multiplex PCR for the detection of ETEC and *E. coli* by targeting the genes *elt* and *est,* as well as the *E. coli* specific stress protein gene *uspA,* whereas Grant et al. (Grant *et al.* 2001) have reported the use of multiplex PCR for the detection of STEC and *E. coli* by targeting *stx*_{*1*}*, stx*_{*2*} and *gadAB.* However, both Osek and Grant are using multiplex PCR targeting a smaller indicator group compared to what is done according to the present invention.

Enterobacteriaceae has been proposed for the replacement of the currently used faecal coliform (FC) indicator group in the microbial quality assessment of water. The definition of the FC indicator group, and which species to include in it, has been the focus of much debate. Coliform isolation methods were often used to define this group albeit the lack of a rational taxonomic basis and none of the coliforms can function as reliable markers for all enteric pathogens (Leclerc *et al.* 2001). In contrast to FC, that was created to fit the human concept of indicator/index group, the family Enterobacteriaceae is a consistent and well-defined phylogenic entity, which is easier to define than the FC indicator group. Also, the choice of Enterobacteriaceae makes it possible to include the detection of important water-born pathogens such as *Salmonella* or *Shigella,* which are not detected by the FC microbiological tests currently in use. Thus, the use of Enterobacteriaceae would constitute a safer indicator marker for assessing the efficiency of food and water treatment. Furthermore, while *E. faecalis* and *E. faecium* both commonly were considered to be harmless commensal of the human digestive tract, they have recently emerged as important aetiological agents for various nosocomial diseases. Aside from virulence factors they have acquired a steadily increasing pool of resistance determinants to antibiotics (ampicillin, aminoglycosides and glycopeptides) which turned them into resilient potentially life threatening pathogenic bacteria. Thus, similar to *E. coli,* they have cumulated relevant traits as being the major members of traditional indicator groups important for water surveillance, and are potential pathogens important to be identified in the clinical world. With regard to virulence factors, the detection of intimin and the shiga encoding genes are considered important, as they are highly associated with the onset of human diarrhoea caused by e.g. the EHEC, EPEC and *Shigella dysenteriaeae* bacteria. Furthermore, as many species or sub-species of bacteria often share similar genes for the coding of specific virulence factors (e.g. *stx* and *eae*), and because these virulence factors often are located on mobile elements, there exist a need for new primers/methods to detect these specific virulence genes in various bacteria groups.

It is therefore an object of the present invention to provide new primers, as well as use of the primers in a method, in order to detect relevant bacteria indicator groups, as well as virulence factors, useful for both clinical diagnostic and for the sanitary evaluation of goods for human consumption. Furthermore, it is also an object of the present invention to enable the detection of new emerging bacteria with specific virulence genes. These objects have been obtained by the present invention, characterized by the enclosed claims.

The present invention relates to several new, oligonucleotide primers and universal degenerate oligonucleotide primers as defined in the claims for the detection of any of three important bacterial indicator groups used in food microbiology, as well as two virulence factors which are associated with the aetiology of several types of watery and bloody human diarrhoea. The three target indicator groups are 1) the Enterobacteriaceae, which includes the total coliform group, 2) the species *E. coli,* a member of the Enterobacteriaceae family, and 3) the two species *E. faecalis* and *E. faecium* belonging to the faecal enterococci indicator group. The target virulence factors are Shiga toxin (Stx) encoded by the gene *stx* and Intimin encoded by the gene *eae.* These two virulence factors are found in EPEC and EHEC among others, and recognized as the two main virulence factors associated with the onset of the human diarrhoea symptoms by these bacterial pathogens. Shiga toxin is found in *Shigella dysenteriae* and has been identified in emerging pathogens such as *Shigella sonnei* and *Citrobacter rodentium,* whereas Intimin has been identified in emerging pathogens such as *Hafnia alvei.* The universal primers according to the present invention were designed in an effort to enable detection of all gene variants of the virulence factors, independently of the bacteria hosting them. Hence, the primers according to the present invention enable the possible detection of new emerging pathogenic bacteria.

The present invention also relates to use of the primers according to the present invention, wherein the primers have the sequences as defined in the claims.

The present invention further discloses the association of indicator markers with virulence markers in a robust and reliable multiplex PCR for the targets of interest wherein, compared to currently used technology, indicator markers also useful for clinical applications are used. Thus, according to another aspect, the present invention also relates to use of the primers according to the present invention, in a method preferably based on multiplex PCRs (i.e. triplex PCR or multiplex PCR depending on the objectives of the analysis and the amount of information needed) which enables the detection of any of three important bacterial indicator groups used in food microbiology and the two virulence factors which are associated with the aetiology of several types of watery and bloody human diarrhoea, as well as the possible detection of new emerging pathogenic bacteria. However, and according to a further aspect of the present invention, simplex PCR may also, depending upon the analysis requirements, be used. Furthermore, and according to the present invention, the method comprise preferably a restriction enzyme digestion and a seminested duplex PCR protocol for the accurate analysis of the *stx* gene.

The present invention will now be described in more detail, with reference to figures and examples. However, the examples are not to be interpreted as restrictive to the scope of the enclosed claims.
Figure 1. Comparison of the alignments of previously described primers aiming at the detection of *stx* genes and their variants; +, forward primer; -, reverse primer; arrows are also indicating the direction of the primers.
Figure 2. Comparison of the alignments of previously described primers for the detection of *eae* genes and their variants; +, forward primer; -, reverse primer; arrows are also indicating the direction of the primers.
Figure 3. Agarose (1,7%) gel electrophoresis of mixtures of DNA templates showing the specificity of the multiplex reactions and the optimization of MgCl₂ and primer concentrations. Lanes: 1, negative control; 2 & 15, DNA size markers; 3, 5, 7, 9, 11 and 13, *E. coli* O157:H7; 4, 6, 8, 10, 12 and 14, *Shigella dysenteriae;* 3 to 8, 0,1 µM eae28UU18/eae748LU21 primers, 0,2 µM UstxU1/ UstxL1 primers and 0,02 µM Meca202UU20/Meca633LU21 primers; 9 to 14, 0,1 µM eae28UU18/eae748LU21 primers, 0,5 µM UstxU1/ UstxL1 primers and 0,05 µM Meca202UU20/Meca633LU21 primers.
Figure 4. Agarose (1,7%) gel electrophoresis of mixtures of DNA templates showing the specificity of the multiplex reactions and the optimization of MgCl₂ concentrations. Lanes: 2, negative control; 3 & 14, DNA size markers; 4, 7 and 10, *E. coli* O157:H7; 5, 8 and 11, *E. coli* O157:H7 with *Shigella dysenteriae;* 6, 9 and 12, *Shigella dysenteriae.*
Figure 5. Agarose (3%) gel electrophoresis showing stx simplex PCR and subsequent Bsr I digestion for 14 different STECs strains and 3 *Shigella dysenteriae* serotype 1. The detailed typing results are shown in Table 9. Lanes 1, 16, 17 & 32, DNA size markers; 2 & 3, *E. coli* O128:H?; 4 & 5, *E. coli* O113:H21; 6 & 7, *E. coli* O157:H7; 8 & 9, *E. coli* O157:H7; 10 & 11, *E. coli* O?:H?; 12 & 13, *Shigella dysenteriae;* 14 & 15, *Shigella dysenteriae;* 18 & 19, *Shigella dysenteriae;* 20 & 21, *E. coli* O157:H7; 22 & 23, *E. coli* O157:H-; 24 & 25, *E. coli* O157:H?; 26 & 27, *E. coli* O157:H7; 28 & 29, *E. coli* O157:H7; 30 & 31, *E. coli* O157:H7;
Figure 6A. Agarose (1,7%) gel electrophoresis showing the specificity of the triplex PCR reactions using 15 different bacterial strains. Lanes: 1, DNA size markers; 2, *E. coli* O128:H?; *3, E. coli* O113:H21; 4, *E. coli* O157:H7; 5, *E. coli* O157:H7; 6, *E. coli* O?:H?; 7, *Shigella dysenteriae;* 8, *Shigella dysenteriae;* 9, *Shigella dysenteriae;* 10, *E. coli* O157:H7; 11, *E. coli* O157:H-; 12, *E. coli* O157:H?; 13, *E. coli* O157:H7; 14, *E. coli* O157:H7; 15, *E. coli* O157:H7; 16, *E. coli* O157:H7.
Figure 6B. Agarose (1,7%) gel electrophoresis showing results from a seminested duplex PCR performed on diluted aliquots of product of triplex PCR shown on Figure 6A.
Figure 6C. Agarose (1,7%) gel electrophoresis showing results from a seminested duplex PCR performed directly on the same strains used for the triplex PCR shown in Figure 6A
Figure 7. Agarose (1,7%) gel electrophoresis of mixtures of DNA templates showing the specificity of the simplex PCR using the Meca479UU21 and Meca722LU21 primer pair. Lanes: 2, DNA size markers; 3, *E. coli* environmental isolate; 4, *E. coli* environmental isolate; *5, Pseudomonas aeruginosa; 6, Pseudomonas fluorescence; 7, E. coli* O26:K60 (EPEC); 8, *E. coli* O44:K74 (EPEC); 9, *Aeromonas hydrophila;* 10, *Aeromonas sobria;* 11, *Shigella flexneri* serotype 2B; 12, *Shigella sonnei;* 13, *Enterococcus faecalis*; 14, *Enterococcus faecium*; 15, *Shigella dysenteriae* serotype 1; 16, *Shigella dysenteriaea* serotype 1.
Figure 8. Agarose (1.7%) gel electrophoresis of simplex stx optimization for MgCl₂ and annealing temperature (Tₐ). Lane 1, DNA size markers; Lanes 2 to 16, EHEC O113:H21; Lanes 2, 5, 8, 11 and 14, 1.5 mM MgCl₂; Lanes 3, 6, 9, 12 and 15, 2 mM MgCl₂; Lanes 4, 7, 10, 13 and 16, 3 mM MgCl₂; Lanes 2, 3 and 4, 47.0°C Tₐ; Lanes 5, 6 and 7, 48.5°C Tₐ; Lanes 8, 9 and 10, 52.1°C Tₐ; Lanes 11, 12 and 13, 54.0°C Tₐ; Lanes 14, 15 and 16, 57.8°C Tₐ; All PCR reactions have 0.1 µM Ustx primer and 0.01 µM stx2f primers.
Figure 9. Alignment of different *stx* primers/sequences.
Figure 10. Alignment of different *eae* primers/sequences.

The present invention relates to oligonucleotide primers for the detection and partial characterisation of relevant bacteria useful for both clinical diagnostic and for the sanitary evaluation of goods for human consumption, in particular water. It has a DNA-based approach to identify some virulence factors associated with the aetiology of human diarrhoea, as well as species and family. Since different species or sub-species share similar genes for the coding of the specific virulence factors, the conserved parts of the sequence of some virulence genes were used to develop PCR primers universal for the targeted pathogenic trait (i.e. universal primers). Sequence variability within the PCR product obtained by use of the primers according to the present invention, can then be exploited for identification of the species or sub-species.

The choosing of either simplex PCR or multiplex PCR will depend upon the objectives of the analysis, as well as the amount of information needed. Routine microbiological control of food, and in particular water, will preferably (only) use the multiplex PCR, i.e. the quadruplex PCR protocol according to one embodiment of the present invention (see example 2), for detecting bacterial indicators, although positive samples might further be analysed with the triplex PCR (see example 1) to assess the presence of virulence factors. When analysing clinical samples, the multiplex PCR, i.e. the triplex PCR protocol according to another embodiment of the present invention, would be chosen for samples originating from patients with diarrhoea, although the quadruplex PCR might be required in other situations where *E. faecium* or *E. faecalis* is suspected and needs to be differentiated from Enterobacteriaceae. Thus, when desired, a more refined analysis using another test sample from the same source can be used for a second round of identification after a positive first round in order to obtain more information. Furthermore, the *stx* PCR product, may be further analysed by for example using a specific set of nested primers (to perform a second multiplex PCR using the product of the first PCR), specific probes or restriction enzymes. According to the present invention, restriction enzymes are used for this sub-typing, preferably after a simplex or triplex PCR amplification comprising the *stx* gene, (see example 3). According to the present invention, a set of seminested primers are used to differentiate stx₁ from stx₂ either after a simplex PCR amplification of the *stx* gene or a triplex PCR comprising the amplification of the *stx* gene or directly from a sample (see example 4).

The virulence genes coding for the chosen pathogenic traits are located on, or associated with, mobile elements which favours inter-species horizontal transfer and the emergence of new pathogens. One of the goals according to the present invention is that the "universal" mode of design of the primers, using degenerate rather than mismatched primers, will provide a tool for the monitoring of these mobile virulence elements thus making possible their detection, including new variants, in previously unknown bacterial hosts. The various bacterial groups and specific virulence genes targeted by use of the primers according to the present invention are presented in Table1. All 42 *stx* sequences and 14 *eae* sequences used in this work are shown in Figures 9 and 10 respectively

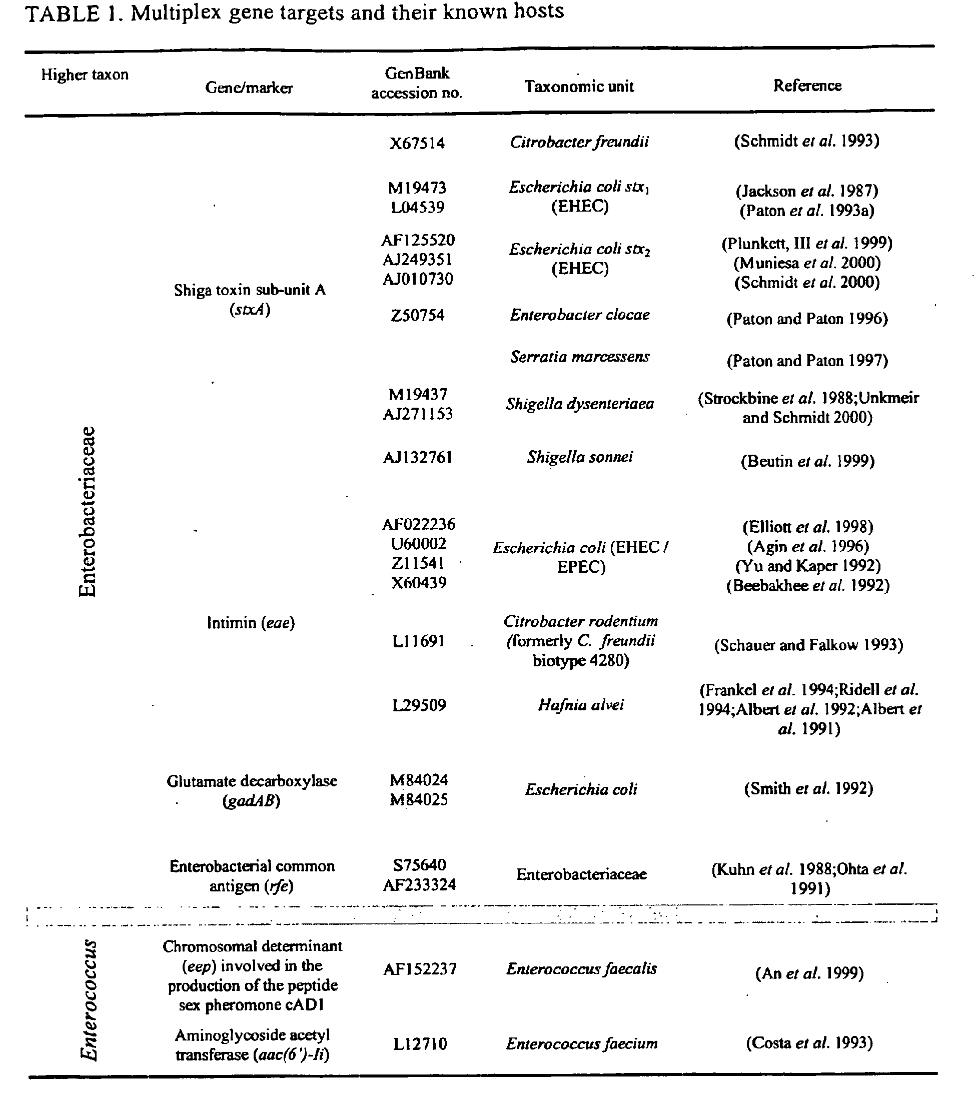

As it can be seen from Table 1, the largest taxonomic unit to be detected by the primers according to the present invention is the bacterial family Enterobacteriaceae. Another important indicator group is the faecal enterococci, including the two major species of importance to humans *E. faecalis* and *E. faecium,* which are specifically detected. Furthermore, the primers according to the present invention was also developed to detect all variations of the genes encoding Intimin and Shiga toxin, since these factors are associated with the onset of human diarrhoea caused by EPEC, EHEC, *Shigella dysenteriaeae* and various emerging pathogenic bacteria. In particular, these virulence factors have been recognized as the most important for the onset of clinical symptoms in humans infected by STEC (Law 2000).

In the present invention, and as it can be seen from Table 1, the genes *rfe* (implicated in the synthesis of ECA), *eae* and *stxA* were used to develop universal primers for the specific detection of the Enterobacteriaceae group by PCR. According to the knowledge of the present inventors, the use of the gene *rfe* in order to detect Enterobacteriaceae has not been described previously. When included in the multiplex PCR, the *rfe* pair of primers will have the advantage of also acting as a positive control for the PCR reaction in some experimental conditions, such as when testing a sample known to contain *E. coli* (i.e. faeces sample). Furthermore, even though the two genes *stx* and *eae* have been extensively used to develop PCR protocols, a need to develop universal primers for clinical diagnostic has been reported in the literature. In addition, the universal primers developed for *stx* (Ustx) (i.e. the A subunit encoding gene of *stx*) and *eae* according to the present invention, have the advantage of being more robust than the few ones already described in the literature, and were designed to work in a multiplex PCR. Also, the use of only one primer pair for the detection of all *stx* variants (which is the case with the universal primers according to the present invention), as opposed to 2 or 3 primer pairs which is the case in the currently used technology, increases the robustness of the multiplex PCR, as well as the possibility of including more targets to the method. Furthermore, the unique association of Enterobacteriaceae as an indicator marker with virulence markers is useful to help bridge the notions of indicator and index markers.

According to the present invention the chromosomal determinant *eep* (An *et al.* 1999) (involved in the production of the peptide sex pheromone cAD1 which induces a mating response from *E. faecalis* donors carrying the haemolysin/bacteriocin (cytolysin) plasmid pAD1) was used to develop primers for the specific detection of *E. faecalis* (Table 1), and according to the knowledge of the present inventors, the *eep* determinant has never been used previously in a PCR protocol as a target for identification of *E. faecalis.* Various sex pheromone are produced for the specific induction of mating response by donors carrying the corresponding specific plasmid, but all sex pheromones appear to be regulated by the *eep* chromosomal determinant. Also, and in accordance with the present invention, another chromosome located antibiotic resistance gene for the specific detection of *E. faecium* has been used, the aminoglycoside acetyl transferase *aac(6')-li* (Table 1) (Costa *et al.* 1993), which confers intrinsic low resistance to aminoglycosides (kanamycin, gentamycin).

Two authors, Lin Z. and Read S.C. (Lin *et al.* 1993;Read *et al.* 1992) have developed PCR methods which use a forward primer overlapping part of the forward Ustx used by the present inventors but use different reverse primers, thus generating amplicons of a different length. Moreover the overlapping forward primers they use have two mismatched positions, whereas the present inventors use degenerate, as well as longer primers. These last two points are important when evaluating robustness of the method and use of these primers in a multiplex PCR. Furthermore, Lin and Read performed only simplex PCR.

Karch (Karch and Meyer 1989) developed a simplex PCR which was later included in multiplex PCR protocols (Fratamico *et al.* 1993;Fratamico and Strobaugh 1998; Nagano *et al.* 1998) in which the reverse primer is complementary to a part of the Ustx forward primer of the present inventors. Again, two mismatches are observed with the Karch primer as shown in Figure 1. Paton et al. in the simplex PCR they developed, (Paton *et al.* 1993b), used an overlapping degenerate perfect match to the reverse primer, although they used a different forward primer. Finally, Yamasaki S. described a simplex PCR (Kobayashi *et al.* 2001;Yamasaki *et al.* 1996) in which both forward and reverse primers are overlapping those of the present technology thus generating an amplicon of almost the same size (5 nucleotide less). The aim and the claim of all these protocols are to detect all variants of the *stx* gene using a single pair of primers. Few studies have compared and tested extensively such protocols, but two publications (Bastian *et al.* 1998) among which a study of the latest gene variant *stx*_{2f} (Schmidt *et al.* 2000), agree in finding that only Lin (Lin *et al.* 1993) achieves detection of all variants. A closer study of the reverse primers used by Paton and Yamasaki (Paton *et al.* 1993b;Yamasaki *et al.* 1996) indicates they would probably also fail because of a on nucleotide insert in the *stx*_{2f} variant symbolised by the gap in Figure 1. To compensate for this gap we have, according to the present invention, extended the notion of degenerate primers by adding an overlapping primer pair with a perfect match to the *stx*_{2f} variant. Although it looks like an extra pair of primers, it is only one more combination to cover all the possible ambiguous positions for the same primer pair location on the *stx* sequence.

All the primers according to the present invention may also be used in simplex PCR protocols, and in particular the primers designed for detecting Enterobacteriaceae, *E. faecium* and *E. faecalis.* Also, all primers according to the present invention were designed using the open reading frames of the targeted genes which allows the technology to be used with RNA based amplification techniques such as NASBA (Organon Technika).

The new primers and universal degenerate primers according to the present invention were designed with the help of Oligo 6 (Molecular Biology Insights, Inc. USA) software for windows and/or designed manually using the alignments results. Primers and degenerate universal primers were designed to enhance unknown variant detection and integrate them in multiplex PCR protocol. All primers designed and used in the method according to the present invention were compared with primers described previously in the literature. Results for *stxA* and *eae* gene alignments, along with primers used in the method according to the present invention and primers previously described in the literature, are shown in Figures 9 and 10. A summary of Figure 9 to compare the Ustx (i.e. universal stx) degenerate primer pair with the most relevant primer pairs used in other PCR protocols, also aiming at the detection of all stx variants with a single pair of primers, is shown in Figure 1. In a similar way, Figure 2 summarises the most relevant previously described primers for the detection of the *eae* gene.

Optimization of the triplex PCR developed to simultaneously detect Enterobacteriaceae and the presence or absence of any variants of *stxA* and *eae* genes are presented in Figure 3 and 4. Various primer pairs were designed according to their potential compatibility in a multiplex PCR, and special care was given to the design of the degenerate primers. Simplex PCR for all primer pairs were first optimized independently by varying physical and chemical conditions such as annealing temperature and primer concentration and were then tested for specificity. Multiplex PCR was then optimized varying annealing temperature, primer concentration and also by testing various additives or facilitators such as DMSO, glycerol, bovine serum albumin, formamide and MgCl₂ which are reported helpful for multiplex PCR. We found that only an increase of MgCl₂ improved the reaction as shown in Figures 3 and 4 although the other PCR facilitators may still be helpful when analysing complex samples (i.e. faeces). A list of primers designed and used in connection with the method according to the present invention is presented in Table 2. The international nomenclature for ambiguous bases used for the degenerate positions in the primers is shown in Table 3.

**TABLE 2. List of primers**

| **Gene** | **Primers (5'-3')** | | **SEQ ID** | **Ta °C** | **Location** | **Amp(bp)** |
|---|---|---|---|---|---|---|
| *stxA*₁ *& stxA*₂*,* universal | UstxU1 | TRTTGARCRAAATAATTTATATGTG | 1 | 52.1 | 279-303* | 526-7 (*stxA*₁) |
| | UstxL1 | MTGATGATGRCAATTCAGTAT | 2 | | 784-805* | 523-4 (*stxA*₂) |
| | UstxL2 | CMTGATGATGRCAATTCAGTAT | 3 | | 783-806* | |
| *stxA*_{2f} | UstxU3 | AATGGAACGGAATAACTTATATGT | 4 | 49.0 | 279-303* | |
| | UstxL3 | GGTTGAGTGGCAATTAAGGAT | 5 | | 784-804* | 523 |
| *stxA*₁ | nestx1U | GTACAACACTKGATGATCTC | 31 | 49.6 | 327-347* | |
| | UstxL1 | MTGATGATGRCAATTCAGTAT | 2 | | 784-805* | 200 |
| *stxA*₂ | nestx2U | TGACRACGGACAGCAGT | 32 | 54.3 | 114-130* | |
| | UstxL1 | MTGATGATGRCAATTCAGTAT | 2 | | 784-805* | 410 |
| | | | | | | |
| *eae* intimin | eae626UU21 | ATTATGGAACGGCAGAGGTTA | 6 | 68 | 626-647* | |
| | eae812LU21 | TGAAGACGTTATAGCCCAACA | 7 | | 812-833* | 207 |
| | eae626UU21 | ATTATGGAACGGCAGAGGTTA | 6 | 60 | 626-647* | |
| | eae956LU21 | GGCGCTCATCATAGTCTTTCT | 8 | | 956-977* | 351 |
| | eae28UU18 | ACCCGGCACAAGCATAAG | 9 | 53.4 | 28-45* | |
| | eae748LU21 | CGTAAAGCGRGAGTCAATRTA | 10 | | 748-768* | 741 |
| | eae28UU18 | ACCCGGCACAAGCATAAG | 9 | 51.8 | 28-45* | |
| | eae956LU21 | GGCGCTCATCATAGTCTTTCT | 11 | | 956-977* | 949 |
| | eae28UU18 | ACCCGGCACAAGCATAAG | 9 | 54.0 | 28-45* | |
| | eae812LU21 | TGAAGACGTTATAGCCCAACA | 7 | | 812-833* | 805 |
| | | | | | | |
| *rfe* for ECA specific for Enterobacteriaceae | Meca479UU21 | TGGATATGGTGGCGATTATGT | 12 | 53.2 | 479-499 | |
| | Meca722LU18 | TCCAGGCMCGCTTAATGC | 13 | | 722-739 | 261 |
| | Meca722LU21 | CYTTCCAGGCMCGCTTAATGC | 14 | | 722-742 | 264 |
| | | | | 55 | | |
| | Meca582UU18 | TTCCCGYCAGGCRTTTGT | 15 | | 582-599 | |
| | Meca826LU21 | CMGGYAWTGGTTGTGTCATCR | 16 | | 826-846 | 265 |
| | Meca202UU20 | GGGTTRTCCWGCGTCTCRTT | 17 | 58.6 | 202-223 | |
| | Meca633LU21 | TATTCTGCCRKYACGCCWAYK | 18 | | 633-653 | 452 |
| | | | | | | |
| *gadA & gadB* glutamate decarboxylase of *E.coli* | gad259U21 | AAAGAAGAATATCCGCAATCC | 19 | 55 | 259-279 | |
| | gad402L17 | GCCATTTCATCGCCATC | 20 | | 402-418 | 160 |
| | gad658U19 | CCACAACCGCTGCACGATG | 21 | 60 | 658-676 | |
| | gad772L21 | CAGGCGGAAGTCCCAGACGAT | 22 | | 772-792 | 135 |
| | | | | | | |
| *eep,* Chromosomal gene involved in the production of the peptide sex pheromone cAD1 of *Enterococcus faecalis* | efam1U | AATGCCGTGGGTAATGTGGTT | 23 | 60 | 855-875 | |
| | efam1L | GGCTTTTCGGGGTTCTTCTG | 24 | | 1329-1348 | 494 |
| | | | | | | |
| | efam2U | TTGAGTTAAATGCCGTGGGTA | 25 | 53 | 257-277 | |
| | efam2L | CATGGGTCCCGCAAAG | 26 | | 525-540 | 284 |
| | | | | | | |
| *aac(6')-li,* chromosomal aminoglycoside acetyl | efuaac1U | GGGGGAAGACGTATGATAATC | 27 | 56 | 191-211 | |
| | efuaac 1L | TCGGGAGCTTTCTACAACTAA | 28 | | 428-448 | 258 |
| transferase of *Enterococcus faecium* | efuaac2U | GGCGTATTTAACTTAGTCGT | 29 | 58 | 1257-1276 | |
| | efuaac2L | TTTGCGTCTTCTCGTAATTT | 30 | | 1449-1468 | 212 |

| | | | | | | |
|---|---|---|---|---|---|---|
| * Numeration is done using the longest hypothetical gene obtained after alignment of all variants (see Figures 6 and 7) | | | | | | |

**TABLE 3. Ambiguous bases nomenclature**

| Symbol | Meaning |
|---|---|
| B | Not A |
| D | Not C |
| H | Not G |
| I | Inosine |
| K | G or T |
| M | A or C |
| N | A or C or G or T |
| R | A or G |
| S | C or G |
| V | Not T |
| W | A or T |
| Y | C or T |

According to Table 2, the following primers correspond to the following sequences:
UstxU1 corresponds to SEQ ID NO 1
UstxL1 corresponds to SEQ ID NO 2
UstxL2 corresponds to SEQ ID NO 3
UstxU3 corresponds to SEQ ID NO 4
UstxL3 corresponds to SEQ ID NO 5
eae626UU21 corresponds to SEQ ID NO 6
eae812LU21 corresponds to SEQ ID NO 7
eae956LU21 corresponds to SEQ ID NO 8
eae28UU 18 corresponds to SEQ ID NO 9
eae748LU21 corresponds to SEQ ID NO 10
eae956LU21 corresponds to SEQ ID NO 11
Meca479UU21 corresponds to SEQ ID NO 12
Meca722LU 18 corresponds to SEQ ID NO 13
Meca722LU21 corresponds to SEQ ID NO 14
Meca582UU18 corresponds to SEQID NO 15
Meca826LU21 corresponds to SEQID NO 16
Meca202UU20 corresponds to SEQ ID NO 17
Meca633LU21 corresponds to SEQ ID NO 18
gad259U21 corresponds to SEQ ID NO 19
gad402L17 corresponds to SEQ ID NO 20
gad658U19 corresponds to SEQ ID NO 21
gad772L21 corresponds to SEQ ID NO 22
efam 1U corresponds to SEQ ID NO 23
efam1L corresponds to SEQ ID NO 24
efam2U corresponds to SEQ ID NO 25
efam2L corresponds to SEQ ID NO 26
efuaac1U corresponds to SEQ ID NO 27
efuaac1L corresponds to SEQ ID NO 28
efuaac2U corresponds to SEQ ID NO 29
efuaac2L corresponds to SEQ ID NO 30
nestx1U corresponds to SEQ ID NO 31
nestx2U corresponds to SEQ ID NO 32

As multiplex PCR methods have been previously developed for the detection of various virulence factors associated with human gastrointestinal diseases, in particular *stxAB*₁*, stxAB*₂ and variants, *eae, hlyA, ipaH* and *eltAB,* an extensive literature search have been performed to look into redundancy with prior art and is presented in Tables 4 and 5.

**TABLE 4 Relevant PCR protocols for the detection of virulence factors and indicator groups**

| Reference | Pathogenic markers | | | | | | | | Serotyping | | | | Indicator marker | | | | | |
|---|---|---|---|---|---|---|---|---|---|---|---|---|---|---|---|---|---|---|
| | *stx*_{*1*} | *stx*_{*2*} | All *stx* | *eae* | *hlyA* | *elt AB* | *est* | *invA* | *eae* | *rf*b | *fli* | *uidA* | *uspA* | *UidA*/*R* | *Gad AB* | *lamB* | *lacZ* | 16S rRNA |
| (Bej *et al.* 1991b; Bej *et al.* 1990; Bej *et al.* 1991a) | | | | | | | | | | | | | | + | | + | + | |
| (Bellin *et al.* 2001) | + | + | | | | | | | | | | | | | | | | |
| (Call *et al.* 2001) | + | + | | + | + | | | | | | | | | | | | | |
| (Carroll *et al.* 2000) | | | | | | | | | | | | | | | | | | + |
| (Cebula *et al.* 1995) | + | + | | | | | | | | | | + | | | | | | |
| (Chen and Griffiths 1998) | | | | | | | | | | | | | + | | | | | |
| (Chen and Griffiths 2001) | + | + | | | | | | + | | | | | | | | | | |
| (China *et al.* 1996) | + | + | | + | | | | | | | | | | | | | | |
| (Desmarchelier *et al.* 1998) | | | | | | | | | | + | | | | | | | | |
| (Fach *et al.* 2001) | | | + | | | | | | | | | | | | | | | |
| (Fagan *et al.* 1999) | + | + | | | + | | | | + | | | | | | | | | |
| (Feng and Monday 2000) | + | + | | + | + | | | | | | | + | | | | | | |
| (Fratamico and Strobaugh 1998) | | | + | | + | | | + | + | | | | | | | | | |
| (Fratamico *et al.* 2000) | + | + | | + | + | | | | | | + | | | | | | | |
| (Fratamico *et al.* 1993) | | | + | | + | | | | + | | | | | | | | | |
| (Gannon *et al.* 1997) | + | + | | | | | | | + | | + | | | | | | | |
| (Gannon *et al.* 1992) | + | + | | | | | | | | | | | | | | | | |
| (Grant *et al.* 2001) | + | + | | | | | | | | | | | | | + | | | |
| (Heuvelink *et al.* 1995) | + | + | | + | | | | | | | | | | | | | | |
| (Karch *et al.* 1993) | | | | + | | | | | | | | | | | | | | |
| (Karch and Meyer 1989) | | | + | | | | | | | | | | | | | | | |
| (Klausegger *et al.* 1999) | | | | | | | | | | | | | | | | | | + |
| (Lang *et al.* 1994) | + | + | | | | + | | | | | | | | | | | | |
| (Lin *et al.* 1993) | | | + | | | | | | | | | | | | | | | |
| (McDaniels *et al.* 1996) | | | | | | | | | | | | | | | + | | | |
| (McDaniels *et al.* 1996) | | | | | | | | | | | | | | + | | | | |
| (Meng *et al.* 1996;Meng *et al.* 1997) | | | | + | | | | | | | | | | | | | | |
| (Mittelman *et al.* 1997) | | | | | | | | | | | | | | | | | | + |
| (Nagano *et al.* 1998) | | | + | | | | | | | + | + | | | | | | | |
| (Olsvik *et al.* 1991;Olsvik and Strockbine 1993) | + | + | | | | | | | | | | | | | | | | |
| (Osek 2001) | | | | | | + | + | | | | | | + | | | | | |
| (Pass *et al.* 2000) | + | + | | + | | | | | | | | | | | | | | |
| (Paton and Paton 1998) | + | + | | + | + | | | | | | | | | | | | | |
| (Paton *et al.* 1993b) | | | + | | | | | | | | | | | | | | | |
| (Paton and Paton 1998) | | | | | | | | | | + | | | | | | | | |
| (Pollard *et al.* 1990) | + | + | | | | | | | | | | | | | | | | |
| (Radu *et al.* 2001) | + | + | | | | | | | | | + | | | | | | | |
| (Read *et al.* 1992) | | | + | | | | | | | | | | | | | | | |
| (Tsen and Jian 1998) | + | + | | | | + | + | | | | | | | | | | | |
| (Yamasaki *et al.* 1996) | | | + | | | | | | | | | | | | | | | |

**TABLE 5. Relevant PCR protocols for detection of E. faecalis and E. faecium**

| Target strains, Patent | PCR | Reference & primer name | Target gene(s) | Sequence (5'-3') | Size of product (bp) |
|---|---|---|---|---|---|
| *Enterococcus* & Universal, | PCR (nested) | (Ke *et al.* 1999), Universal degenerate U1 & U2 Ent1 & Ent2 | *tuf,* chromosomal, elongation factor EF-Tu | AAYATGATIACIGGIGCIGCICARATGGA AYRTTTCCCGGCATIACCAT | 803 |
| | | | | TACTGACAAACCATTCATGATG | |
| | | | | TACTGACAAACCATTCATGATG | |
| | | | | AACTTCGTCACCAACGCGAAC | 112 |
| *Enterococcus durans, Enterococcus hirae* | Multipplex (2), | (Knijff *et al.* 2001), DuHifF, DuR & HiR | *ddl,* chromosomal D-Ala D-Ala ligase for peptidoglycan final synthesis step (sensitive to glycopeptides) | TTATGTCCCWGTWTTGAAAAATCAA TGAATCATATTGGTATGCAGTCCG | 186 |
| | | | | TTTTGTTAGACCTCTTCCGGA | 377 |
| *Enterococcus faecalis Enterococcus faecium* | Probe technology | (Betzl *et al.* 1990), DB8 | 23S rRNA | TAGGTGTTGTTAGCATTTCG | |
| | | DB6 | | CACACAATCGTAACATCCTA | |
| *Enterococcus faecium* / *faecalis - glycopeptide resistance* | Cycle Probe Technology (CPT), 5' terminal labeled with [γ-³²P]-ATP. | (Modrusan *et al.,* 1999), van*A*811L-27 Van*B*467-27 | *VanA* and *VanB-B2* glycopeptide resistance genes glycopeptide resistance genes | TTAATAACCCAAAAGGCGGGAGTAGCT | |
| | | | | TACATTCTTACAAAAAATGCGGGCATC | |
| *Enterococcus faecium* | PCR (1) | (Cheng *et al.* 1997), EM1A & B *et al.* 1997), EM1A & | Randomly selected species specific *E. faecium* DNA sequence, unknown function unknown function | TTGAGGCAGACCAGATTGACG | |
| | | | | TATGACAGCGACTCCGATTCC | 658 |
| Enterococcus faecium / US5,994,066 | PCR (1) | (Bergeron *et al.* 1999) | *sod ,* superoxide dismutase | ACGCAACAATGGTGGTGGACA | |
| | | | | TCTTGATTTGCAGTAGAGGTAATAG | |
| *Enterococcus gallinarum* | Multiplex (3) | (Clark *et al.* 1998) (Angeletti *vanC-1, et al.* 2001) (Satake *et al.* 1997) | D-Ala D-Ser ligase for peptidoglycan final synthesis step (constitutive low resistance to glycopeptides) | GAAAGACAACAGGAAGACCGC | 796 |
| | | | | ATCGCATCACAAGCACCAATC | |
| *Enterococcus casseliflavus* | | vanC2-1 & -2 | *vanC-2* | CGGGGAAGATGGCAGTAT | |
| | | | | CGCAGGGACGGTGATTTT | 484 |
| *Enterococcus flavescens* | | | *vanC-3,* | GCCTTTACTTATTGTTCC | |
| | | | | GCTTGTTCTTTGACCTTA | 224 |
| *Enterococcus - glycopeptide resistance* | Multipplex (2) | (Petrich *et al.* 1999), VanA1& VanA2 | *vanA*, D-Ala D-Lac ligase for peptidoglycan final synthesis step (aquired high resistance to vancomycin & teicoplanin) | Biotin-GCTGCGATATTCAAAGCTCA CAGTACAATGCGGCCGTTA | 545 |
| | EIA detection in microtiter plates with anti-FITC HRP conjugate | VanA3 | | ATTGCGTAGTCCAATTC-Fluorescein | |
| | | (Petrich *et al.* 1999) (Dutka-Malen *et al.* 1995), VanB1&VanB3 | *vanB,* D-Ala D-Lac ligase for peptidoglycan final synthesis step (aquired high resistance to vancomycin) | Biotin-ATGGGAAGCCGATAGTC GTTACGCCAAAGGACGAAC | 368 |
| | | VanB4 | | GACAATTCAAACAGACC-Fluorescein | |
| *Enterococcus -* glycopeptide resistance | Multiplex (6) | (Dutka-Malen *et al.* 1995) / A, & A₂, (Roger *et al.* 1999) | *vanA,* D-Ala D-Lac ligase for peptidoglycan final synthesis step (aquired high resistance to vancomycin & teicoplanin) | GGGAAAACGACAATTGC | 732 |
| | | (Angeletti *et al.* 2001) (Satake *et al.* 1997) | *vanB,* D-Ala D-Lac ligase for peptidoglycan final synthesis step (aquired high resistance to vancomycin) | GTACAATGCGGCCGTTA | |
| | | B₁ & B₂ (Isenberg 1998) | | ATGGGAAGCCGATAGTC | |
| | | | | GATTTCGTTCCTCGACC | 635 |
| *Enterococcus gallinarum* | | p662, C₁ & C₂ (Dutka-Malen *et al.* 1995) / (Isenberg 1998) p662 | *vanC-1,* D-Ala D-Ser ligase for peptidoglycan final synthesis step (constitutive low resistance to glycopeptides) | GGTATCAAGGAAACCTC | |
| | | | | CTTCCGCCATCATAGCT | 822 |
| *Enterococcus casseliflavus & flavescens* | | D₁ & D₂ | *vanC-2* & *vanC-3,* D-Ala D-Ser ligase for peptidoglycan final synthesis step (constitutive low resistance to glycopeptides) | CTCCTACGATTCTCTTG | |
| | | | | CGAGCAAGACCTTTAAG | 439 |
| *Enterococcus faecalis* | | E₁ & E₂ | *ddl*_{*E.faecalis*}, chromosomal D-Ala D-Ala ligase for peptidoglycan final synthesis step (sensitive to glycopeptides) | ATCAAGTACAGTTAGTCT | |
| | | | | ACGATTCAAAGCTAACTG | 941 |
| *Enterococcus faecium* | | F₁ & F₂ | *ddl*_{*E.faecium*}*,* chromosomal D-Ala D-Ala ligase for peptidoglycan final synthesis step (sensitive to glycopeptides) | TAGAGACATTGAATATGCC | 550 |
| | | | | TCGAATGTGCTACAATC | |
| *Enterococcus* species | PCR, tRNA intergenic spacer PCR, capillary electrophoresis of amplicons | (Baele *et al.* 2000), T5A & T3B*(TET) | tRNA genes flanking conserved edges | AGTCCGGTGCTCTAACCAACTGAG | variable |
| | | | | AGGTCGCGGGTTCGAATCC | |
| Enterococcus with species Srtreptococcus species & Gram positive bacteria | PCR (broad range) degenerate primers, sequencing of the *al.* 1995), product and comparison to a database | (Poyart *et al.* 2000) (Poyart *et* d1 & d2 (Poyart *et al*. 1998) | *sod*, superoxide dismutase Universal degenerated primers for the amplification of a 480 Nu internal fragment *sodA*_{*int*} | CCITAYICITAYGAYGCIYTIGARCCARRT | 480 |
| | | | | ARTAIGCRTGYTCCCAIACRTC | |

### Best mode

Triplex PCR: For sample analysis (i.e. the detection of bacteria), individual colonies (i.e. test samples) from Luria agar culture plates are suspended in sterile distilled water. The preparation is then either used for direct PCR of bacteria or first boiled 10 min before use for the PCR. Samples (10 µl) were amplified in 50 µl final reaction mixtures using a BioTest Biometra PCR machine. The mixtures contained 0,1 mM each dATP, dCTP, dGTP, dUTP, 1X final concentration of the 10X buffer solution and 1 U of DyNazyme II (Finnzymes) DNA polymerase per reaction. MgCl₂ is adjusted from 1.5 mM standard concentration in the 1X buffer to 3 mM final concentration. Primer concentrations are as following, 0.05 µM for Meca202UU20 and Meca633LU21; 0.5 µM for UstxU1; 0,3 µM for UstxL1; 0.02 µM for UstxU3 and UstxL3 (The triplex is not as robust when using UstxU3 and UstxL3 and should therefore not be added if the subtype *stx*_{*2f*} is not researched); and 0.15 µM for eae28UU18 and eae748LU21. The PCR conditions consisted of 2 min preheating at 94°C for one cycle followed by 15 s denaturation at 94°C, 30 s annealing at 57°C and 60 s at 72°C for 40 cycles; and 5 min final elongation at 72°C. Reaction products are separated by agarose (1,7%) gel electrophoresis with 0,5X Tris-borate-EDTA buffer and stained with ethidium bromide 0.5 µg/ml. PCR products are separated by applying seventy-five volts and 25 mA across the gel for about 1 h 30 min.

Quadruplex PCR: For direct sample analysis (i.e. the detection of bacteria) of water, the sample is centrifuged at 12000 rpm for 5 min and the pellet is re-suspended in sterile distilled water two times before it is re-suspended in a 50µl final volume when PCR inhibitors are expected to be found. The preparation is then boiled 10 min before use for the PCR. Samples (10 µl) were amplified in 50 µl final reaction mixtures using a BioTest Biometra PCR machine. The mixtures contained 0,1 mM each dATP, dCTP, dGTP, dUTP, 1X final concentration of the 10X buffer solution and 1 U of DyNazyme II (Finnzymes) DNA polymerase per reaction. MgCl₂ is adjusted from 1.5 mM standard concentration in the 1X buffer to 3 mM final concentration. Primer concentrations are as following, 0.5 µM for efam 1U and efam 1L; 0.05 µM for Meca582UU18 and Meca826LU21; 0.5 µM for efuaac2U and efuaac2L; and 0.5 µM for gad259U21 and gad402L17. The PCR conditions consisted of 2 min preheating at 94°C for one cycle followed by 15 s denaturation at 94°C, 30 s annealing at 57°C and 60 s at 72°C for 40 cycles; and 5 min final elongation at 72°C. Reaction products are separated by agarose (1,7%) gel electrophoresis with 0,5X Tris-borate-EDTA buffer and stained with ethidium bromide 0.5 µg/ml. PCR products are separated by applying seventy-five volts and 25 mA across the gel for about 1 h 30 min.

BsrI endonuclease restriction: The restriction endonucleases Bsr I with the recognition site ACTGG(1/-1) was purshased at New England Biolab. Digestion was performed using 16 µl PCR product (from stx simplex PCR or tiplex PCR), 10 U BsrI with the provided NEB3 buffer 1X final in a total volume of 20µl. PCR tubes were used and digestion was carried out at 65°C for 2h30min in the thermocycler. Reaction products are separated by agarose (3%) gel electrophoresis with 0,5X Tris-borate-EDTA buffer and stained with ethidium bromide 0.5 µg/ml. PCR products are separated by applying seventy-five volts and 25 mA across the gel for about 1 h.

Stx seminested duplex PCR: For typing (stx₁ / stx₂) the product of a first positive simplex or triplex PCR (as described in Examplel). The PCR product must be diluted to 10⁻³ before 5 µl sample is used in a 50 µl final reaction mixtures using a BioTest Biometra PCR machine. The mixtures contained 0,1 mM each dATP, dCTP, dGTP, dUTP, 1X final concentration of the 10X buffer solution and 1 U of DyNazyme II (Finnzymes) DNA polymerase per reaction. MgCl₂ is adjusted from 1.5 mM standard concentration in the 1 X buffer to 3 mM final concentration. Primer concentrations are as following 0.1 µM for UstxL1, 0.3 µM for nestx1U and 0.05 µM for nestx2U. The PCR conditions consisted of 2 min preheating at 94°C for one cycle followed by 15 s denaturation at 94°C, 30 s annealing at 57°C and 60 s elongation at 72°C for 30 cycles; and 5 min final elongation at 72°C. Reaction products are separated by agarose (1,7%) gel electrophoresis with 0,5X Tris-borate-EDTA buffer and stained with ethidium bromide 0.5 µg/ml. PCR products are separated by applying seventy-five volts and 25 mA across the gel for about 1 h. The method can also be used directly on DNA samples.

### EXAMPLES

### Example 1. Simultaneous detection of stx and eae virulence genes and Enterobacteriaceae in a multiplex (triplex) PCR

For sample analysis (i.e. the detection of bacteria), individual colonies (i.e. test samples) from Luria agar culture plates are suspended in sterile distilled water. The preparation is then either used for direct PCR of bacteria or first boiled 10 min before use for the PCR. Samples (10 µl) are amplified in 50 µl final reaction mixtures using a BioTest Biometra PCR machine. The mixture contains 0,1 mM each dATP, dCTP, dGTP, dUTP, 1X final concentration of the 10X buffer solution and 1 U of DyNazyme II (Finnzymes) DNA polymerase per reaction. Concentrations for MgCl₂ and primers were optimized for each multiplex and are shown in Table 6 and 7. The PCR conditions consists of 2 min preheating at 94°C for one cycle followed by 15 s denaturation at 94°C, 30 s annealing at 57°C and 60 s elongation at 72°C for 40 cycles; and 5 min final elongation at 72°C. Reaction products are separated by agarose (1,7%) gel electrophoresis stained with ethidium bromide (0.5 µg/ml), and the results are shown in Figure 3 and 4. 0,5X Tris-borate-EDTA buffer is used for the electrophoresis.

Seventy-five volts and 25 mA are applied across the gel for about 1 h 30 min to separate the PCR products.

**TABLE 6. Sequences of primers, conditions to perform the triplex PCR and product sizes.**

| Gene | Primers (5'-3') | | | Location | Product size (bp) | Primers µM | MgCl₂ mM | Ta °C |
|---|---|---|---|---|---|---|---|---|
| *rfe* for ECA | Meca202UU20 | SEQ ID NO 17 | GGGTTRTCCWGCGTCTCRTT | 202-223 | 452 | 0.05 | 3 | 57 |
| | Meca633LU21 | SEQ ID NO 18 | TATTCTGCCRKYACGCCWAYK | 633-653 | | | | |
| *stxA*₁ *&* | UstxU1 | SEQ ID NO 1 | TRTTGARCRAAATAATTTATATGT | 279-303* | 526 (*stxA*₁) | 0.5 | | |
| *stxA*₂, universal | UstxL1 | SEQ ID NO 2 | MTGATGATGRCAATTCAGTAT | 784-805* | 523 (*stxA*₂) | 0.3 | | |
| *stxA*_{2f} | UstxU3 | SEQ ID NO 4 | AATGGAACGGAATAACTTATATGT | 279-303* | 523 (*stxA*_{2f}) | 0.02 | | |
| | UstxL3 | SEQ ID NO 5 | GGTTGAGTGGCAATTAAGGAT | 784-804* | | | | |
| *eae* | eae28UU18 | SEQ ID NO 9 | ACCCGGCACAAGCATAAG | 28-45* | 741 | 0.15 | | |
| intimin | eae748LU21 | SEQ ID NO 10 | CGTAAAGCGRGAGTCAATRTA | 748-768* | | | | |

| | | | | | | | | |
|---|---|---|---|---|---|---|---|---|
| * Numeration is done using the longest hypothetical gene obtained after alignment of all variants (see Figures 9 and 10). | | | | | | | | |

The results show that only an increase in the concentration of MgCl₂ improved the reaction with an optimum concentration reached at 3mM. Similarly optimum concentrations for the primers as indicated in Table 6 was demonstrated as shown in Figure 3 where column 9 to 14 have optimum primer concentration. Furthermore, specificity is demonstrated in Figure 4, as only the samples containing *E. co*li O157H7 produces the expected band of 741 bp that indicates the presence of the intimin gene *eae.* Finally, samples containing either *E. co*li O157H7 or *Shigella dysenteriaea* both show amplification products for the *stx* and *rfe* gene as expected.

### Example 2. Simultaneous detection of Enterobacteriaceae, E. coli, E. faecalis and E. faecium in a multiplex (quadruplex) PCR

The quadruplex PCR operational characteristics are similar to those of the triplex described in example 1 apart from specific conditions given in Table 7.

**TABLE 7. Sequences of primers, conditions to perform the quadruplex PCR and product sizes.**

| Gene | Primers (5'-3') | | | Location | Product size (bp) | Primers µM | MgCl₂ mM | Ta °C |
|---|---|---|---|---|---|---|---|---|
| *eep,* Chromosomal | efam1U | SEQ ID NO 23 | AATGCCGTGGGTAATGTGGTT | 855-875 | 494 | 0.5 | 3 | 55 |
| gene of *E. faecalis* | efam1L | SEQ ID NO 24 | GGCTTTTCGGGGTTCTTCTG | 1329-1348 | | | | |
| *rfe* for ECA | Meca582UU18 | SEQ ID NO 15 | TTCCCGYCAGGCRTTTGT | 582-599 | 265 | 0.050.05 | | |
| | Meca826LU21 | SEQ ID NO 16 | CMGGYAWTGGTTGTGTCATCR | 826-846 | | | | |
| *aac(6')-Ii,* chromosomal | efuaac2U | SEQ ID NO 29 | GGCGTATTTAACTTAGTCGT | 1257-1276 | 212 | 0.5 | | |
| gene of *E. faecium* | efuaac 2L | SEQ ID NO 30 | TTTGCGTCTTCTCGTAATTT | 1449-1468 | | | | |
| *gadAB* of *E. coli* | gad259U21 | SEQ ID NO 19 | AAAGAAGAATATCCGCAATCC | 259-279 | 160 | 0.5 | | |
| | gad402L17 | SEQ ID NO 20 | GCCATTTCATCGCCATC | 402-418 | | | | |

### Example 3. Sub-typing of stx by endonuclease restriction of stx PCR amplification product

The restriction endonucleases HaeII, HindIII and BsrI, purchased at New England Biolab, were chosen to obtain the appropriate restriction patterns as shown in Table 8. Endonuclease restriction is performed on the product of simplex PCR using Ustx primers (see example 6).

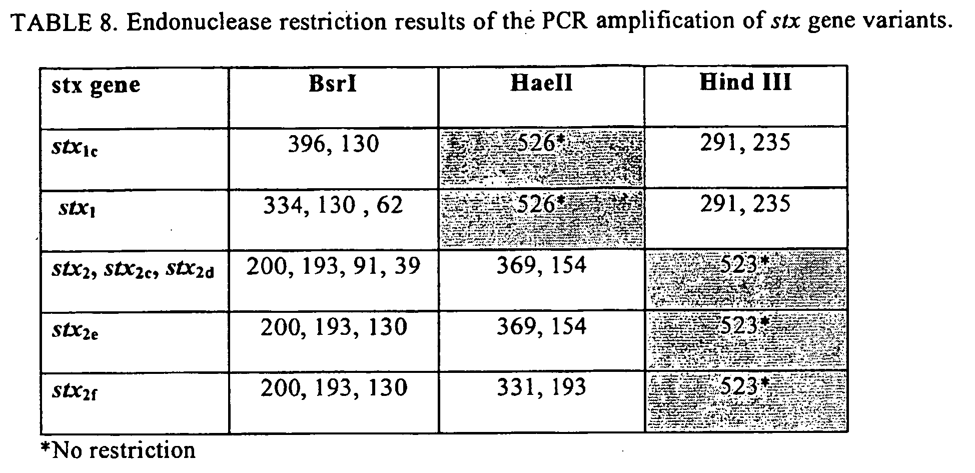

Results are shown in Figure 5 and Table 9 for BsrI endonuclease restriction of the *stx* PCR amplicon. 0,5X Tris-borate-EDTA buffer is used for the electrophoresis. Seventy-five volts and 25 mA are applied across the gel for about 1 h 30 min to separate the digestion products. When using PCR products from the triplex amplification the *eae* amplicon (741 bp) is discriminated in two groups. Amplicons from *eae* α, β, δ and ε will give two fragments of 88 and 654 bp while *eae* γ will give three fragments of 88, 178 and 476 bp.

**TABLE 9. Summary of results shown in Figure 5 (BsrI restriction of stx amplification)**

| **Lane on Fig. 5** | **Species** | ***stxA*** | ***stxA***_{**1c**} | ***stxA***_{**1**} | **All *stxA***_{**2**} | **All stx *- stxA***_{**2**} | ***stxA***_{**2,2c,2d**} | **Results** |
|---|---|---|---|---|---|---|---|---|
| | | **523-526** | **396** | **334** | **200-193** | **130** | **91** | |
| 2 & 3 | *E. coli* EHEC O128:H? | + | + | - | + | + | + | *stxA*_{1c}*+ stxA*_{2,2c,2d} |
| 4 & 5 | *E. coli* EHEC O113:H21 | + | - | + | + | + | + | *stxA*₁ + *stxA*_{2,2c,2d} |
| 6 & 7 | EHEC *E*. *coli* O157:H7 | + | - | + | + | + | + | |
| 8 & 9 | *E. coli* EHEC O157:H7 | + | - | + | - | + | - | *stxA*₁ |
| 10 & 11 | *E. coli* EHEC O?:H? | + | - | + | - | + | - | |
| 12 & 13 | *Shigella dysenteriae* Serotype 1 | + | - | + | - | + | - | |
| 14 & 15 | *Shigella dysenteriae* Serotype 1 | + | - | + | - | + | - | |
| 17 & 18 | *Shigella dysenteriae* Serotype 1 | + | - | + | - | + | - | |
| 19 & 20 | *E. coli* EHEC O157:H7 | + | - | - | + | - | + | *stxA*_{2,2c,2d} |
| 21 & 22 | *E. coli* EHEC O157:H- | + | - | - | + | - | + | |
| 23 & 24 | *E. coli* EHEC O157:H? | + | - | - | + | - | + | |
| 25 & 26 | *E. coli* EHEC O157:H7 | + | - | - | + | - | + | |
| 27 & 28 | *E. coli* EHEC O157:H7 | + | - | - | + | - | + | |
| 29 & 30 | *E. coli* EHEC O157:H7 | + | - | - | + | - | + | |

### Example 4. stx Seminested duplex PCR to differentiate stx1 and stx2 after a simplex PCR, triplex PCR or directly

The seminested duplex PCR operational characteristics are similar to those of the triplex described in example 1 apart from specific conditions given in Table 10. The method can be used on 10⁻³ diluted aliquots from a triplex stx PCR as shown in Figure 6A and 6B or directly from individual colonies prepared as indicated in example 1 (see results in Figure 6C). This method is very sensitive and 25 cycles are sufficient when using aliquots from a triplex PCR. The results shown in Figure 6B and 6C corroborate those obtained using the BsrI endonuclease restriction method shown in Figure 5 and Table 9.

**TABLE 10. Sequences of primers, conditions to perform the seminested duplex PCR and product sizes**

| Gene | Primer sequence (5'-3') | | | Location | Product size (bp) | Primers µM | MgCl₂ mM | Ta °C |
|---|---|---|---|---|---|---|---|---|
| *stxA*₁ *& stxA*₂ | UstxL1 | SEQ ID NO 2 | MTGATGATGRCAATTCAGTAT | 784-805* | | 0.1 | 3 | 57 |
| *stxA*₁ | Nestx1 | SEQ ID NO 31 | GTACAACACTKGATGATCTC | 327-347* | 200 | 0.3 | | |
| *StxA*₂ | Nestx2 | SEQ ID NO 32 | TGACRACGGACAGCAGT | 114-130* | 410 | 0.05 | | |

| | | | | | | | | |
|---|---|---|---|---|---|---|---|---|
| * Numeration is done using the longest hypothetical gene obtained after alignment of all variants (see Figures 9 and 10). | | | | | | | | |

### Example 5. Simplex PCR example for the detection of Enterobacteriaceae

For sample analysis (i.e. the detection of bacteria), individual colonies (i.e. test samples) from Luria agar culture plates are suspended in sterile distilled water. The preparation is then either used for direct PCR of bacteria or first boiled 10 min before use for the PCR. Samples (10 µl) are amplified in 50 µl final reaction mixtures using a BioTest Biometra PCR machine. The mixtures contains 0,1 mM each dATP, dCTP, dGTP, dUTP, 1X final concentration of the 10X buffer solution and 1 U of DyNazyme II (Finnzymes) DNA polymerase per reaction and 0.1 µM of each primer Meca479UU21 and Meca722LU21. The PCR conditions consists of 2 min preheating at 94°C for one cycle followed by 15 s denaturation at 94°C, 30 s annealing at 57°C and 60 s elongation at 72°C for 40 cycles; and 5 min final elongation at 72°C. Reaction products are separated by agarose (1,7%) gel electrophoresis stained with ethidium bromide (0.5 µg/ml), and the results are shown in Figure 6. 0,5X Tris-borate-EDTA buffer is used for the electrophoresis. One hundred volts and 40 mA are applied across the gel for about 50 min to separate the PCR products.

Simplex PCR using Meca479UU21 and Meca722LU21 is performed on various Enterobacteriaceae and non-Enterobacteriaceae species to illustrate specificity of the method. As shown in Figure 7, no non-Enterobacteriaceae was amplified by the simplex PCR.

### Example 6. Simplex PCR example for the detection of stx

For sample analysis (i.e. the detection of bacteria), individual colonies (i.e. test samples) from Luria agar culture plates are suspended in sterile distilled water. The preparation is then either used for direct PCR of bacteria or first boiled 10 min before use for the PCR. Samples (10 µl) are amplified in 50 µl final reaction mixtures using a BioTest Biometra T gradient PCR machine. The mixtures contains 0,1 mM each dATP, dCTP, dGTP, dUTP, 1X final concentration of the 10X buffer solution and 1 U of DyNazyme II (Finnzymes) DNA polymerase per reaction and 0.1 µM of each primer UstxU1 and UstxL1, and 0.01 µM of each primer UstxU3 and UstxL3. The PCR conditions consists of 2 min preheating at 94°C for one cycle followed by 15 s denaturation at 94°C, 30 s annealing at 57°C and 60 s elongation at 72°C for 40 cycles; and 5 min final elongation at 72°C. Reaction products are separated by agarose (1,7%) gel electrophoresis stained with ethidium bromide (0.5 µg/ml), and the results are shown in Figure 8. 0,5X Tris-borate-EDTA buffer is used for the electrophoresis. One hundred volts and 40 mA are applied across the gel for about 1h.

**TABLE 11. Sequences of primers and optimum conditions to perform the simplex stx PCR and product sizes.**

| Gene | Primers (5'-3') | | | Location | Product size (bp) | Primers µM | MgCl₂ mM | Ta°C |
|---|---|---|---|---|---|---|---|---|
| *stxA*₁ & | UstxU1 | SEQ ID NO 1 | TRTTGARCRAAATAATTTATATGT | 279-303* | 526 (*stxA*₁) | 0.1 | 3 | 57 |
| *stxA*₂, universal | UstxL1 | SEQ ID NO 2 | MTGATGATGRCAAITCAGTAT | 784-805* | 523 (*stxA*₂) | 0.1 | | |
| *stxA*_{2f} | UstxU3 | SEQ ID NO 4 | AATGGAACGGAATAACTTATATGT | 279-303* | 523 (*stxA*_{2f}) | 0.01 | | |
| | UstxL3 | SEQ ID NO 5 | GGTTGAGTGGCAATTAAGGAT | 784-804* | | | | |

Results shown in Figure 8 demonstrate how important MgCl₂ concentration is to develop a robust PCR amplification.

### References A

1. An F. Y., Sulavik M. C., & Clewell D. B. (1999) Identification and Characterization of a Determinant (eep) on the Enterococcus faecalis Chromosome That Is Involved in Production of the Peptide Sex Pheromone cAD1. *The Journal of Bacteriology* 181: 5915-5921.
2. Bastian S. N., Carle I., & Grimont F. (1998) Comparison of 14 PCR systems for the detection and subtyping of stx genes in Shiga-toxin-producing Escherichia coli. *Research in Microbiology* 149: 457-472.
3. Bej A. K., Mahbubani M. H., Dicesare J. L., & Atlas R. M. (1991) Polymerase chain reaction-gene probe detection of microorganisms by using filter-concentrated samples. *Applied and Environmental Microbiology* 57: 3529-3534.
4. Bergeron M. G., Picard F. J., Ouellette M., & Roy P. H. Species-specific and universal DNA probes and amplification primers to rapidly detect and identify common bacterial pathogens and associated antibiotic resistance genes from clinical specimens for routine diagnosis in microbiology laboratories. US PTO 5,994,066. 1999. Ref Type: Patent
5. Betzl D., Ludwig W., & Schleifer K. H. (1990) Identification of lactococci and enterococci by colony hybridization with 23S rRNA-targeted oligonucleotide probes. *Applied and Environmental Microbiology* 56: 2927-2929.
6. Cheng S., McCleskey F. K., Gress M. J., Petroziello J. M., Liu R., Namdari H., Beninga K., Salmen A., & DelVecchio V. G. (1997) A PCR assay for identification of Enterococcus faecium. *Journal of Clinical Microbiology* 35: 1248-1250.
7. Costa Y., Galimand M., Leclercq R., Duval J., & Courvalin P. (1993) Characterization of the chromosomal aac(6')-Ii gene specific for Enterococcus faecium. *Antimicrobial Agents and Chemotherapy 37 :* 1896-1903.
8. Dutka-Malen S., Evers S., & Courvalin P. (1995) Detection of glycopeptide resistance genotypes and identification to the species level of clinically relevant enterococci by PCR [published erratum appears in J Clin Microbiol 1995 May;33(5):1434]. *Journal of Clinical Microbiology* 33: 24-27.
9. Feng P., Lum R., & Chang G. W. (1991) Identification of uidA gene sequences in beta-D-glucuronidase-negative Escherichia coli. *Applied and Environmental Microbiology* 57: 320-323.
10. Fratamico P. & Strobaugh T. P. (1998) Simultaneous detection of Salmonella spp and Escherichia coli O157:H7 by multiplex PCR. *Journal of Industrial Microbiology & Biotechnology* 21: 92-98.
11. Fratamico P. M., Bhaduri S., & Buchanan R. L. (1993) Studies on *Escherichia coli* serotype O157:H7 strains containing a 60-MDa plasmid and on 60-MDa plasmid-cured derivates. *Journal of Medical Microbiology* 39: 371-381.
12. Grant M. A., Weagant S. D., & Feng P. (2001) Glutamate Decarboxylase Genes as a Prescreening Marker for Detection of Pathogenic Escherichia coli Groups. *Applied and Environmental Microbiology* 67: 3110-3114.
13. Hubner I., Steinmetz I., Obst U., Giebel D., & Bitter-Suermann D. (1992) Rapid determination of members of the family Enterobacteriaceae in drinking water by an immunological assay using a monoclonal antibody against enterobacterial common antigen. *Applied and Environmental Microbiology* 58: 3187-3191.
14. Karch H. & Meyer T. (1989) Single primer pair for amplifying segments of distinct Shiga-like-toxin genes by polymerase chain reaction. *Journal of Clinical Microbiology* 27: 2751-2757.
15. Ke D., Picard F. J., Martineau F., Menard C., Roy P. H., Ouellette M., & Bergeron M. G. (1999) Development of a PCR assay for rapid detection of enterococci [In Process Citation]. *Journal of Clinical Microbiology* 37: 3497-3503.
16. Kobayashi H., Shimada J., Nakazawa M., Morozumi T., Pohjanvirta T., Pelkonen S., & Yamamoto K. (2001) Prevalence and Characteristics of Shiga Toxin-Producing Escherichia coli from Healthy Cattle in Japan. *Applied and Environmental Microbiology* 67: 484-489.
17. Kuhn H. M., Meier-Dieter U., & Mayer H. (1988) ECA, the enterobacterial common antigen. *FEMS Microbiology Reviews* 4: 195-222.
18. Kunin C. M. (1963) Separation, characterization, and biological significance of a common antigen in *Enterobacteriaceae. J.Exp.Med.* 541-548.
19. Law D. (2000) Virulence factors of Escherichia coli O157 and other Shiga toxin-producing E. coli. *Journal of Applied Microbiology* 88: 729-745.
20. Leclerc H., Mossel D. A. A., Edberg S. C., & Struijk C. B. (2001) ADVANCES IN THE BACTERIOLOGY OF THE COLIFORM GROUP: Their Suitability as Markers of Microbial Water Safety. *Annual Review of Microbiology* 55: 201-234.
21. Lin Z., Kurazono H., Yamasaki S., & Takeda Y. (1993) Detection of Various Variant Verotoxin Genes in *Escherichia coli* by Polymerase Chain Reaction. *Microbiology And Immunology* 37: 543-548.
22. Malkamaki M. (1981) Antibodies to the enterobacterial common antigen: standardization of the passive hemagglutination test and levels in normal human sera. *Journal of Clinical Microbiology* 13: 1074-1079.
23. McDaniels A. E., Rice E. W., Reyes A. L., Johnson C. H., Haugland, RA, & Stelma G. N., Jr. (1996) Confirmational identification of Escherichia coli, a comparison of genotypic and phenotypic assays for glutamate decarboxylase and beta-D-glucuronidase. *Applied and Environmental Microbiology* 62: 3350-3354.
24. Mittelman M. W., Habash M., Lacroix J.-M., Khoury A. E., & Krajden (1997) Rapid detection of Enterobacteriaceae in urine by fluorescent 16S rRNA in situ hybridization on membrane filters. *Journal of Microbiological Methods* 30: 153-160.
25. Nagano 1., Kunishima M., Itoh Y., Wu Z., & Takahashi Y. (1998) Detection of verotoxin-producing Escherichia coli O157:H7 by multiplex polymerase chain reaction. *Microbiol.Immunol.* 42: 371-376.
26. Ohta M., Ina K., Kusuzaki K., Kido N., Arakawa Y., & Kato N. (1991) Cloning and expression of the rfe-rff gene cluster of Escherichia coli. *Molecular Microbiology* 5: 1853-1862.
27. Osek J. (2001) Multiplex polymerase chain reaction assay for identification of enterotoxigenic Escherichia coli strains. *J. Vet.Diagn.Invest* 13: 308-311.
28. Paton A. W., Paton J. C., Goldwater P. N., & Manning P. A. (1993) Direct detection of Escherichia coli Shiga-like toxin genes in primary fecal cultures by polymerase chain reaction. *Journal of Clinical Microbiology* 31: 3063-3067.
29. Public Health Laboratory Services (1994) The Microbiology of Water 1994 Part 1-Drinking Water. Report on Public Health and Medical Subjects No. 71. Methods for the examination of waters and associated materials., Sixth edition, Second impression 1995 edn. HMSO Books.
30. Read S. C., Clarke R. C., Martin A., De Grandis S. A., Hii J., McEwen S., & Gyles C. L. (1992) Polymerase chain reaction for detection of verocytotoxigenic Escherichia coli isolated from animal and food sources. *Molecular & Cellular Probes* 6: 153-161.
31. Schmidt H., Scheef J., Morabito S., Caprioli A., Wieler L. H., & Karch H. (2000) A new Shiga toxin 2 variant (Stx2f) from Escherichia coli isolated from pigeons. *Appl.Environ.Microbiol* 66: 1205-1208.
32. World Health Organization. Zoonotic non-O157 Shiga toxin-producing Escherichia coli (STEC). Report of a WHO scientific working group meeting. WHO/CSR/APH/98.8. 1998.
   Ref Type: Report
33. Yamasaki S., Lin Z., Shirai H., Terai A., Oku Y., Ito H., Ohmura M., Karasawa T., Tsukamoto T., Kurazono H., & Takeda Y. (1996) Typing of verotoxins by DNA colony hybridization with poly- and oligonucleotide probes, a bead-enzyme-linked immunosorbent assay, and polymerase chain reaction. *Microbiol-Immunol.* 40: 345-352.

### References B

1. Agin,T.S., Cantey,J.R., Boedeker,E.C. & Wolf,M.K. (1996) *Characterization of the eaeA gene from rabbit enteropathogenic Escherichia coli strain RDEC-1 and comparison to other eaeA genes from bacteria that cause attaching-effacing lesions. FEMS Microbiology Letters,* **144**, 249-258.
2. Albert,M.J., Alam,K., Islam,M., Montanaro,J., Rahaman,A.S., Haider,K., Hossain,M.A., Kibriya,A.K. & Tzipori,S. (1991) *Hafnia alvei, a probable cause of diarrhea in humans [see comments]. Infection and Immunity,* **59,** 1507-1513.
3. Albert,M.J., Faruque,S.M., Ansaruzzaman,M., Islam,M.M., Haider,K., Alam,K., Kabir,I. & Robins-Browne,R. (1992) *Sharing of virulence-associated properties at the phenotypic and genetic levels between enteropathogenic Escherichia coli and Hafnia alvei. Journal of Medical Microbiology,* **37**, 310-314.
4. An,F.Y., Sulavik,M.C. & Clewell,D.B. (1999) *Identification and Characterization of a Determinant (eep) on the Enterococcus faecalis Chromosome That Is Involved in Production of the Peptide Sex Pheromone cAD1. The Journal of Bacteriology,* **181**, 5915-5921.
5. Angeletti,S., Lorino,G., Gherardi,G., Battistoni,F., De Cesaris,M. & Dicuonzo,G. (2001) *Routine Molecular Identification of Enterococci by Gene-Specific PCR and 16S Ribosomal DNA Sequencing. Journal of Clinical Microbiology,* **39**, 794-797.
6. Baele,M., Baele,P., Vaneechoutte,M., Storms,V., Butaye,P., Devriese,L.A., Verschraegen,G., Gillis,M. & Haesebrouck,F. (2000) *Application of tRNA Intergenic Spacer PCR for Identification of Enterococcus Species. Journal of Clinical Microbiology,* **38**, 4201-4207.
7. Beebakhee,G., Louie,M., de Azavedo,J. & Brunton,J. (1992) *Cloning and nucleotide sequence of the eae gene homologue from enterohemorrhagic Escherichia coli serotype O157:H7. FEMS Microbiology Letters,* **91**, 63-68.
8. Bej,A.K., Dicesare,J.L., Haff,L. & Atlas,R.M. (1991 a) *Detection of Escherichia coli and Shigella spp. in water by using the polymerase chain reaction and gene probes for uid. Applied and Environmental Microbiology,* **57**, 1013-1017.
9. Bej,A.K., Mahbubani,M.H., Dicesare,J.L. & Atlas,R.M. (1991b) *Polymerase chain reaction-gene probe detection of microorganisms by using filter-concentrated samples. Applied and Environmental Microbiology,* **57**, 3529-3534.
10. Bej,A.K., Steffan,R.J., DiCesare,J., Haff,L. & Atlas,R.M. (1990) *Detection of coliform bacteria in water by polymerase chain reaction and gene probes. Applied and Environmental Microbiology,* **56**, 307-314.
11. Bellin,T., Pulz,M., Matussek,A., Hempen,H.G. & Gunzer,F. (2001) *Rapid detection of enterohemorrhagic escherichia coli by real-time PCR with fluorescent hybridization probes. J.Clin Microbiol,* **39**, 370-374.
12. Bergeron, M. G., Picard, F. J., Ouellette, M., and Roy, P. H. Species-specific and universal DNA probes and amplification primers to rapidly detect and identify common bacterial pathogens and associated antibiotic resistance genes from clinical specimens for routine diagnosis in microbiology laboratories. US PTO 5,994,066. 30-11-1999.
   Ref Type: Patent
13. Betzl,D., Ludwig,W. & Schleifer,K.H. (1990) *Identification of lactococci and enterococci by colony hybridization with 23S rRNA-targeted oligonucleotide probes. Applied and Environmental Microbiology,* **56**, 2927-2929.
14. Beutin,L., Strauch,E. & Fischer,I. (1999) *Isolation of Shigella sonnei lysogenic for a bacteriophage encoding gene for production of Shiga toxin. Lancet,* **353**, 1498.
15. Call,D.R., Brockman,F.J. & Chandler,D.P. (2001) *Detecting and genotyping Escherichia coli O157:H7 using multiplexed PCR and nucleic acid microarrays. Int.J.Food Microbiol.,* **67**, 71-80.
16. Carroll,N.M., Jaeger,E.E., Choudhury,S., Dunlop,A.A., Matheson,M.M., Adamson,P., Okhravi,N. & Lightman,S. (2000) *Detection of and Discrimination between Gram-Positive and Gram-Negative Bacteria in Intraocular Samples by Using Nested PCR. Journal of Clinical Microbiology,* **38,** 1753-1757.
17. Cebula,T.A., Payne,W.L. & Feng,P. (1995) *Simultaneous identification of strains of Escherichia coli serotype O157:H7 and their Shiga-like toxin type by mismatch amplification mutation assay-multiplex PCR [published erratum appears in J Clin Microbiol 1995 Apr;33(4):1048]. Journal of Clinical Microbiology,* **33**, 248-250.
18. Chen,J. & Griffiths,M.W. (1998) *PCR differentiation of Escherichia coli from other Gram-negative bacteria using primers derived from the nucleotide sequences flanking the gene encoding the universal stress protein [In Process Citation]. Letters in Applied Microbiology,* **27**, 369-371.
19. Chen,J. & Griffiths,M.W. (2001) *Detection of Salmonella and simultaneous detection of Salmonella and Shiga-like toxin-producing Escherichia coli using the magnetic capture hybridization polymerase chain reaction. Lett Appl.Microbiol,* **32**, 7-11.
20. Cheng,S., McCleskey,F.K., Gress,M.J., Petroziello,J.M., Liu,R., Namdari,H., Beninga,K., Salmen,A. & DelVecchio,V.G. (1997) *A PCR assay for identification of Enterococcus faecium. Journal of Clinical Microbiology,* **35**, 1248-1250.
21. China,B., Pirson,V. & Mainil,J. (1996) *Typing of bovine attaching and effacing Escherichia coli by multiplex in vitro amplification of virulence-associated genes. Applied and Environmental Microbiology,* **62**, 3462-3465.
22. Clark,N.C., Teixeira,L.M., Facklam,R.R. & Tenover,F.C. (1998) *Detection and differentiation of vanC-1, vanC-2, and vanC-3 glycopeptide resistance genes in enterococci [In Process Citation]. Journal of Clinical Microbiology,* **36**, 2294-2297.
23. Costa,Y., Galimand,M., Leclercq,R., Duval,J. & Courvalin,P. (1993) *Characterization of the chromosomal aac(6')-Ii gene specific for Enterococcus faecium. Antimicrobial Agents and Chemotherapy,* **37**, 1896-1903.
24. Desmarchelier,P.M., Bilge,S.S., Fegan,N., Mills,L., Vary,J.C.J. & Tarr,P.I. (1998) A *PCR specific for Escherichia coli O157 based on the rfb locus encoding O157 lipopolysaccharide. Journal of Clinical Microbiology,* **36**, 1801-1804.
25. Dutka-Malen,S., Evers,S. & Courvalin,P. (1995) *Detection ofglycopeptide resistance genotypes and identification to the species level of clinically relevant enterococci by PCR [published erratum appears in J Clin Microbiol 1995 May; 33(5):1434]. Journal of Clinical Microbiology,* **33**, 24-27.
26. Elliott,S.J., Wainwright,L.A., McDaniel,T.K., Jarvis,K.G., Deng, YK, Lai,L.C., McNamara,B.P., Donnenberg,M.S. & Kaper,J.B. (1998) *The complete sequence of the locus of enterocyte effacement (LEE) from enteropathogenic Escherichia coli E2348*/*69. Molecular Microbiology,* **28**, 1-4.
27. Fach,P., Perelle,S., Dilasser,F. & Grout,J. (2001) *Comparison between a PCR-ELISA test and the vero cell assay for detecting Shiga toxin-producing Escherichia coli in dairy products and characterization of virulence traits of the isolated strains. Journal of Applied Microbiology,* **90,** 809-818.
28. Fagan,P.K., Hornitzky,M.A., Bettelheim,K.A. & Djordjevic,S.P. (1999) *Detection of shiga-like toxin (stx1 and stx2), intimin (eaeA), and enterohemorrhagic Escherichia coli (EHEC) hemolysin (EHEC hlyA) genes in animal feces by multiplex PCR. Applied and Environmental Microbiology,* **65**, 868-872.
29. Feng,P. & Monday,S.R. (2000) *Multiplex PCR for detection of trait and virulence factors in enterohemorrhagic escherichia coli serotypes [In Process Citation]. Mol Cell Probes,* **14**, 333-337.
30. Frankel,G., Candy,D.C., Everest,P. & Dougan,G. (1994) *Characterization of the C-terminal domains of intimin-like proteins of enteropathogenic and enterohemorrhagic Escherichia coli, Citrobacter freundii, and Hafnia alvei. Infection and Immunity,* **62**, 1835-1842.
31. Fratamico,P. & Strobaugh, T .P. (1998) *Simultaneous detection of Salmonella spp and Escherichia coli O157:H7 by multiplex PCR. Journal of Industrial Microbiology & Biotechnology,* **21**, 92-98.
32. Fratamico,P.M., Bagi,L.K. & Pepe,T. (2000) *A multiplex polymerase chain reaction assay for rapid detection and identification of Escherichia coli O157:H7 in foods and bovine feces. J.Food Prot.,* **63**, 1032-1037.
33. Fratamico,P.M., Bhaduri,S. & Buchanan,R.L. (1993) *Studies on Escherichia coli serotype 0157:H7 strains containing a 60-MDa plasmid and on 60-MDa plasmid-cured derivates. Journal of Medical Microbiology,* **39**, 371-381.
34. Gannon,V.P., D'Souza,S., Graham,T., King,R.K., Rahn,K. & Read (1997) *Use of the flagellar H7 gene as a target in multiplex PCR assays and improved specificity in identification of enterohemorrhagic Escherichia coli strains. Journal of Clinical Microbiology,* **35**, 656-662.
35. Gannon,V.P., King,R.K., Kim,J.Y. & Thomas,E.J. (1992) *Rapid and sensitive method for detection of Shiga-like toxin-producing Escherichia coli in ground beef using the polymerase chain reaction. Applied and Environmental Microbiology,* **58**, 3809-3815.
36. Gannon,V.P., Rashed,M., King,R.K. & Thomas,E.J. (1993) *Detection and characterization of the eae gene of Shiga-like toxin-producing Escherichia coli using polymerase chain reaction. Journal of Clinical Microbiology,* **31**, 1268-1274.
37. Grant,M.A., Weagant,S.D. & Feng,P. (2001) *Glutamate Decarboxylase Genes as a Prescreening Marker for Detection of Pathogenic Escherichia coli Groups. Applied and Environmental Microbiology,* **67**, 3110-3114.
38. Heuvelink,A.E., Van-De-Kar,N.-C.A., Meis,J.-F.G., Monnens,L.-A.H. & Melchers,W.-J.G. (1995) *Characterization of verocytotoxin-producing Escherichia coli O157 isolates from patients with haemolytic uraemic syndrome in western Europe. Epidemiology & Infection,* **115,** 1-14.
39. Hubbard,A.L., Harrison, D.J., Moyes,C. & McOrist,S. (1998) *Direct detection of eae-positive bacteria in human and veterinary colorectal specimens by PCR [In Process Citation]. Journal of Clinical Microbiology,* **36,** 2326-2330.
40. Isenberg,H.D. (1998) *Essential Procedures for Clinical Microbiology.* American Society for Microbiology.
41. Jackson,M.P. (1991) *Detection of Shiga toxin-producing Shigella dysenteriae type 1 and Escherichia coli by using polymerase chain reaction with incorporation of digoxigenin-11-dUTP. Journal of Clinical Microbiology,* **29**, 1910-1914.
42. Jackson,M.P., Newland,J.W., Holmes,R.K. & O'Brien,A.D. (1987) *Nucleotide sequence analysis of the structural genes for Shiga-like toxin I encoded by bacteriophage 933J from Escherichia coli. Microbial Pathogenesis,* **2**, 147-153.
43. Karch,H., Bohm,H., Schmidt,H., Gunzer,F., Aleksic,S. & Heesemann,J. (1993) *Clonal structure and pathogenicity of Shiga-like toxin-producing, sorbitol-fermenting Escherichia coli O157:H-. Journal of Clinical Microbiology,* **31**, 1200-1205.
44. Karch,H. & Meyer,T. (1989) *Single primer pair for amplifying segments of distinct Shiga-like-toxin genes by polymerase chain reaction. Journal of Clinical Microbiology,* **27**, 2751-2757.
45. Ke,D., Picard,F.J., Martineau,F., Menard, C., Roy,P.H., Ouellette,M. & Bergeron,M.G. (1999) *Development of a PCR assay for rapid detection of enterococci [In Process Citation]. Journal of Clinical Microbiology,* **37**, 3497-3503.
46. Klausegger,A., Hell,M., Berger,A., Zinober,K., Baier,S., Jones,N., Sperl,W. & Kofler,B. (1999) *Gram type-specific broad-range PCR amplification for rapid detection of 62 pathogenic bacteria [published erratum appears in J Clin Microbiol 1999 May;37(5):1660]. Journal of Clinical Microbiology,* **37**, 464-466.
47. Knijff,E., Dellaglio,F., Lombardi,A., Andrighetto,C. & Torriani,S. (2001) *Rapid identification of Enterococcus durans and Enterococcus hirae by PCR with primers targeted to the ddl genes. Journal of Microbiological Methods,* **47**, 35-40.
48. Kobayashi,H., Shimada,J., Nakazawa,M., Morozumi,T., Pohjanvirta,T., Pelkonen,S. & Yamamoto,K. (2001) *Prevalence and Characteristics of Shiga Toxin-Producing Escherichia coli from Healthy Cattle in Japan. Applied and Environmental Microbiology,* **67**, 484-489.
49. Kuhn,H.M., Meier-Dieter,U. & Mayer,H. (1988) *ECA, the enterobacterial common antigen. FEMS Microbiology Reviews,* **4**, 195-222.
50. Lang,A.L., Tsai,Y.L., Mayer,C.L., Patton,K.C. & Palmer, C.J. (1994) *Multiplex PCR for detection of the heat-labile toxin gene and shiga-like toxin I and II genes in Escherichia coli isolated from natural waters. Applied and Environmental Microbiology,* **60**, 3145-3149.
51. Lin,Z., Kurazono,H., Yamasaki,S. & Takeda,Y. (1993) *Detection of Various Variant Verotoxin Genes in Escherichia coli by Polymerase Chain Reaction. Microbiology And Immunology,* **37**, 543-548.
52. McDaniels, A.E., Rice,E.W., Reyes,A.L., Johnson,C.H., Haugland, RA & Stelma,G.N., Jr. (1996) *Confirmational identification of Escherichia coli, a comparison of genotypic and phenotypic assays for glutamate decarboxylase and beta-D-glucuronidase. Applied and Environmental Microbiology,* **62**, 3350-3354.
53. Meng,J., Zhao,S., Doyle,M.P., Mitchell,S.E. & Kresovich,S. (1996) *Polymerase chain reaction for detecting Escherichia coli O157: H7. International Journal of Food Microbiology,* **32**, 103-113.
54. Meng,J., Zhao,S., Doyle,M.P., Mitchell,S.E. & Kresovich,S. (1997) *A multiplex PCR for identifying Shiga-like toxin-producing Escherichia coli O157:H*7 *Letters in Applied Microbiology,* **24**, 172-176.
55. Mittelman,M.W., Habash,M., Lacroix,J.-M., Khoury,A.E. & Krajden (1997) *Rapid detection of Enterobacteriaceae in urine by fluorescent 16S rRNA in situ hybridization on membrane filters. Journal of Microbiological Methods,* **30**, 153-160.
56. Modrusan,Z., Marlowe,C., Wheeler,D., Pirseyedi,M. & Bryan,R.N. (1999) *Detection of vancomycin resistant genes vanA and vanB by cycling probe technology. Mol Cell Probes,* **13**, 223-231.
57. Muniesa,M., Recktenwald,J., Bielaszewska,M., Karch,H. & Schmidt,H. (2000) *Characterization of a Shiga Toxin 2e-Converting Bacteriophage from an Escherichia coli Strain of Human Origin. Infection and Immunity,* **68**, 4850-4855.
58. Nagano,I., Kunishima,M., Itoh,Y., Wu,Z. & Takahashi,Y. (1998) *Detection of verotoxin-producing Escherichia coli O157:H7 by multiplex polymerase chain reaction. Microbiol.Immunol.,* **42**, 371-376.
59. Ohta,M., Ina,K., Kusuzaki,K., Kido,N., Arakawa,Y. & Kato,N. (1991) *Cloning and expression of the rfe-rff gene cluster of Escherichia coli. Molecular Microbiology*, **5**, 1853-1862.
60. Olsvik,O., Rimstad,E., Hornes,E., Strockbine,N., Wasteson,Y., Lund,A. & Wachsmuth,K. (1991) *A nested PCR followed by magnetic separation of amplified fragments for detection of Escherichia coli Shiga-like toxin genes. Molecular & Cellular Probes,* **5,** 429-435.
61. Olsvik,O. & Strockbine,N.A. (1993) PCR detection of heat-stable, heat-labile, and Shiga-like toxin genes in *Escherichia coli. Diagnostic molecular microbiology: principles and applications* (Persing, D. H., Smith, T. F., Tenover, F. C., and White, T. J.), pp. 271-276. American Society for Microbiology, Washington D.C.
62. Osek,J. (2001) *Multiplex polymerase chain reaction assay for identification of enterotoxigenic Escherichia coli strains. J. Vet.Diagn.Invest,* **13**, 308-311.
63. Pass,M.A., Odedra,R. & Batt,R.M. (2000) *Multiplex PCRs for Identification of Escherichia coli Virulence Genes. Journal of Clinical Microbiology,* **38**, 2001-2004.
64. Paton,A.W. & Paton,J.C. (1996) *Enterobacter cloacae producing a Shiga-like toxin II-related cytotoxin associated with a case of hemolytic-uremic syndrome. Journal of Clinical Microbiology,* **34**, 463-465.
65. Paton,A.W. & Paton,J.C. (1998) *Detection and characterization of Shiga toxigenic Escherichia coli by using multiplex PCR assays for stx1, stx2, eaeA, enterohemorrhagic E. coli hlyA, rfbO111, and rfbO157. Journal of Clinical Microbiology,* **36**, 598-602.
66. Paton,A.W., Paton,J.C., Goldwater,P.N., Heuzenroeder,M.W. & Manning,P.A. (1993a) *Sequence of a variant Shiga-like toxin type-I operon of Escherichia coli O111:H-. Gene,* **129**, 87-92.
67. Paton,A.W., Paton,J.C., Goldwater,P.N. & Manning,P.A. (1993b) *Direct detection of Escherichia coli Shiga-like toxin genes in primary fecal cultures by polymerase chain reaction. Journal of Clinical Microbiology,* **31**, 3063-3067.
68. Paton,J.C. & Paton,A.W. (1997) *Instability of a Shiga toxin type 2 gene in Enterobacter cloacae [letter]. Journal of Clinical Microbiology,* **35,** 1917.
69. Petrich,A.K., Luinstra,K.E., Groves,D., Chernesky,M.A. & Mahony,J.B. (1999) *Direct detection of vanA and vanB genes in clinical specimens for rapid identification of vancomycin resistant enterococci (VRE) using multiplex PCR [In Process Citation]. Mol Cell Probes,* **13**, 275-281.
70. Plunkett,G., III, Rose,D.J., Durfee,T.J. & Blattner,F.R. (1999) *Sequence of Shiga Toxin 2aPhage 933W from Escherichia coli O157:H7: Shiga Toxin as a Phage Late-Gene Product. The Journal of Bacteriology,* **181**, 1767-1778.
71. Pollard,D.R., Johnson,W.M., Lior,H., Tyler,S.D. & Rozee,K.R. (1990) *Rapid and specific detection of verotoxin genes in Escherichia coli by the polymerase chain reaction [published erratum appears in J Clin Microbiol 1990 Jun;28(6):1491]. Journal of Clinical Microbiology,* **28,** 540-545.
72. Poyart,C., Berche, P. & Trieu-Cuot,P. (1995) *Characterization of super oxide dismutase genes from gram-positive bacteria by polymerase chain reaction using degenerate primers. FEMS Microbiol Lett,* **131**, 41-45.
73. Poyart,C., Quesne,G., Coulon,S., Berche,P. & Trieu-Cuot,P. (1998) *Identification of streptococci to species level by sequencing the gene encoding the manganese-dependent superoxide dismutase. J.Clin.Microbiol,* **36,** 41-47.
74. Poyart,C., Quesnes,G. & Trieu-Cuot,P. (2000) *Sequencing the gene encoding manganese-dependent superoxide dismutase for rapid species identification of enterococci. J.Clin.Microbiol,* **38**, 415-418.
75. Radu,S., Ling,O.W., Rusul,G., Karim,M.I. & Nishibuchi,M. (2001) *Detection of Escherichia coli O157:H7 by multiplex PCR and their characterization by plasmid profiling, antimicrobial resistance, RAPD and PFGE analyses. Journal of Microbiological Methods,* **46**, 131-139.
76. Read,S.C., Clarke,R.C., Martin,A., De Grandis,S.A., Hii,J., McEwen,S. & Gyles,C.L. (1992) *Polymerase chain reaction for detection of verocytotoxigenic Escherichia coli isolated from animal and food sources. Molecular & Cellular Probes,* **6**, 153-161.
77. Reid,S.D., Betting,D.J. & Whittam,T.S. (1999) *Molecular detection and identification of intimin alleles in pathogenic Escherichia coli by multiplex PCR. Journal of Clinical Microbiology,* **37**, 2719-2722.
78. Ridell,J., Siitonen,A., Paulin,L., Mattila,L., Korkeala,H. & Albert,M.J. (1994) *Hafnia alvei in stool specimens from patients with diarrhea and healthy controls. Journal of Clinical Microbiology,* **32**, 2335-2337.
79. Roger,M., Faucher,M.C., Forest,P., St Antoine,P. & Coutlee,F. (1999) *Evaluation of a vanA-specific PCR assay for detection of vancomycin- resistant Enterococcus faecium during a hospital outbreak. Journal of Clinical Microbiology,* **37**, 3348-3349.
80. Satake,S., Clark,N., Rimland,D., Nolte,F.S. & Tenover,F.C. (1997) *Detection of vancomycin-resistant enterococci in fecal samples by PCR. Journal of Clinical Microbiology,* **35**, 2325-2330.
81. Schauer,D.B. & Falkow,S. (1993) *The eae gene of Citrobacter freundii biotype 4280 is necessary for colonization in transmissible murine colonic hyperplasia. Infection and Immunity,* **61**, 4654-4661.
82. Schmidt,H., Montag,M., Bockemuhl,J., Heesemann,J. & Karch,H. (1993) *Shiga-like toxin II-related cytotoxins in Citrobacter freundii strains from humans and beef samples. Infection and Immunity,* **61,** 534-543.
83. Schmidt,H., Scheef, J., Morabito,S., Caprioli,A., Wieler,L.H. & Karch,H. (2000) *A new Shiga toxin 2 variant (Stx2f) from Escherichia coli isolated from pigeons. Appl.Environ.Microbiol,* **66,** 1205-1208.
84. Smith,D.K., Kassam,T., Singh,B. & Elliott,J.F. (1992) *Escherichia coli has two homologous glutamate decarboxylase genes that map to distinct loci. Journal of Bacteriology,* **174**, 5820-5826.
85. Strockbine,N.A., Jackson,M.P., Sung,L.M., Holmes,R.K. & O'Brien,A.D. (1988) *Cloning and sequencing of the genes for Shiga toxin from Shigella dysenteriae type 1. Journal of Bacteriology,* **170**, 1116-1122.
86. Tsen,H.Y. & Jian,L.Z. (1998) *Development and use of a multiplex PCR system for the rapid screening of heat labile toxin I, heat stable toxin II and shiga-like toxin I and II genes ofEscherichia coli in water. Journal of Applied Microbiology,* **84,** 585-592.
87. Unkmeir,A. & Schmidt,H. (2000) *Structural analysis of phage-borne stx genes and their flanking sequences in shiga toxin-producing Escherichia coli and Shigella dysenteriae type I strains. Infect Immun.,* **68,** 4856-4864.
88. Yamasaki,S., Lin,Z., Shirai,H., Terai,A., Oku,Y., Ito,H., Ohmura,M., Karasawa,T., Tsukamoto,T., Kurazono,H. & Takeda,Y. (1996) *Typing of verotoxins by DNA colony hybridization with poly- and oligonucleotide probes, a bead-enzyme-linked immunosorbent assay, and polymerase chain reaction. Microbiol.Immunol.,* **40,** 345-352.
89. Yu,J. & Kaper,J.B. (1992) *Cloning and characterization of the eae gene of enterohaemorrhagic Escherichia coli O157:H7. Molecular Microbiology,* **6**, 411-417.

## Claims

1. A set of oligonucleotide primers for multiplex PCR comprising primers having the nucleic acid sequence as defined according to:
a) SEQ ID NO:17, SEQ ID NO:18, SEQ ID NO: 1 to 5, SEQ ID NO: 9, SEQ ID NO: 10 and optionally SEQ ID NO: 31 to 32: or
b) SEQ ID NO:15, SEQ ID NO:16, SEQ ID NO:19, SEQ ID NO:20, SEQ ID NO:23, SEQ ID NO:24, SEQ ID NO:29 and SEQ ID NO:30.

2. Method for the detection of bacterial indicator groups and/or virulence factors,
**characterized in** comprising the following steps:
a) amplifying the targeted DNA in a test sample suspected to contain the targeted DNA by the use of a set of oligonucleotide primers according to claim 1 in multiplex PCR processes; and optionally
b) subtyping the *stx* encoded virulence factor by seminested duplex PCR by the use of a set of oligonucleotide primers having the nucleic acid sequences as defined according to SEQ ID NO:1-5, SEQ ID NO:31 and SEQ ID NO:32.

3. Method according to claim 2,
**characterized in that** it comprises amplifying the DNA by using triplex PCR with primers having the nucleic acid sequence as defined according to SEQ ID NO: 1-5, SEQ ID NO:9 and 10 and SEQ ID NO:17 and 18 in order to detect the presence of Enterobacteriaceae as well as the association or not of any of the two virulence genes *stx* and *eae*.

4. Method according to claim 2,
**characterized in that** it comprises amplifying the DNA by using quadruplex PCR with primers comprising oligonucleotides corresponding to SEQ ID NO:15 and 16, SEQ ID NO:19 and 20, SEQ ID NO:23 and 24 and SEQ ID NO:29 and 30, to detect the presence of Enterobacteriaceae, *Escherichia coli, Enterococcus faecalis* and *Enterococcus faecium.*

5. Method according to the claim 2 or claim 3, wherein any of the primers according to SEQ ID NO:1 to SEQ ID NO:5, SEQ ID NO:31 and SEQ ID NO:32 are used to detect all variants of the Shiga toxin genes *stx* by seminested duplex PCR.

## Patentansprüche

1. Gruppe von Oligonukleotid-Primern für die Multiplex-PCR, umfassend Primer mit den wie folgt definierten Nukleinsäuresequenzen:
a) SEQ ID Nr. 17, SEQ ID Nr. 18, SEQ ID Nr. 1 bis 5, SEQ ID Nr. 9, SEQ ID Nr. 10 und gegebenenfalls SEQ ID Nr. 31 bis 32 oder
b) SEQ ID Nr. 15, SEQ ID Nr. 16, SEQ ID Nr. 19, SEQ ID Nr. 20, SEQ ID Nr. 23, SEQ ID Nr. 24, SEQ ID Nr. 29 und SEQ ID Nr. 30.

2. Verfahren zum Nachweis von bakteriellen Indikatorgruppen und/oder Virulenz-Faktoren,
**dadurch gekennzeichnet, dass** es die folgenden Schritte umfasst:
a) Amplifizieren der Ziel-DNA in einer Untersuchungsprobe, die in Verdacht steht, die Ziel-DNA zu enthalten, durch die Verwendung einer Gruppe von Oligonukleotid-Primern nach Anspruch 1 in Multiplex-PCR-Verfahren; und gegebenenfalls
b) Subtypisieren des *stx*-codierten Virulenzfaktors durch Seminested-Duplex-PCR unter Verwendung einer Gruppe von Oligonukleotid-Primern mit den gemäß SEQ ID Nr. 1 bis 5, SEQ ID Nr. 31 und SEQ ID Nr. 32 definierten Nukleinsäuresequenzen.

3. Verfahren nach Anspruch 2,
**dadurch gekennzeichnet, dass** es das Amplifizieren der DNA unter Verwendung von Triplex-PCR umfasst, mit Primern, die eine gemäß SEQ ID Nr. 1 bis 5, SEQ ID Nr. 9 und 10 und SEQ ID Nr. 17 und 18 definierte Nukleinsäuresequenz aufweisen, um das Vorhandensein von *Enterobacteriaceae* sowie die Assoziation oder Nicht-Assoziation der beiden Virulenz-Gene *stx* und *eae* nachzuweisen.

4. Verfahren nach Anspruch 2,
**dadurch gekennzeichnet, dass** es das Amplifizieren der DNA unter Verwendung von Quadruplex-PCR umfasst, mit Primern, die Oligonukleotide entsprechend SEQ ID Nr. 15 und 16, SEQ ID Nr. 19 und 20 und SEQ ID Nr. 23 und 24 und SEQ ID Nr. 29 und 30 umfassen, um das Vorhandensein von *Enterobacteriaceae, Escherichia coli, Enterococcus faecalis* und *Enterococcus faecium* nachzuweisen.

5. Verfahren nach Anspruch 2 oder Anspruch 3, wobei beliebige der Primer gemäß SEQ ID Nr. 1 bis SEQ ID Nr. 5, SEQ ID Nr. 31 und SEQ ID Nr. 32 verwendet werden, um alle Varianten der Shiga Toxin Gene *stx* durch Seminested-Duplex-PCR nachzuweisen.

## Revendications

1. Un groupe d'amorces à oligonucléotides pour PCR multiplex, comprenant les amorces avec les séquences nucléiques définies suivant :
a. SEQ ID NO: 17, SEQ ID NO: 18, SEQ ID NO: 1 à 5, SEQ ID NO: 9, SEQ ID NO: 10 et en option SEQ ID NO: 31 à 32 ou
b. SEQ ID NO: 15, SEQ ID NO: 16, SEQ ID NO: 19 SEQ ID NO: 20, SEQ ID NO: 23, SEQ ID NO: 24, SEQ ID NO: 29 et SEQ ID NO: 30

2. Une méthode pour la détection de groupe bactériens indicateur et / ou facteurs de virulence, **caractérisés par** la possession des étapes suivantes:
a. Amplification de l'ADN ciblé dans un échantillon à tester suspecté de contenir l'ADN ciblé, par l'utilisation d'un groupe d'amorces à oligonucléotides définis par la revendication 1 dans un processus de PCR multiplex ; et en option
b. sous typage du gene *stx* codant pour le facteur de virulence par PCR semi-emboîtée duplex en utilisant un groupe d'amorces à oligonucléotides comprenant les séquences nucléiques définies suivant SEQ ID NO: 1 à 5, SEQ ID NO: 31 et SEQ ID NO: 32

3. Une méthode suivant la revendication 2, **caractérisée par** l'amplification d'ADN en utilisant une PCR triplex avec les amorces ayant les séquences d'acide nucléique définies suivant SEQ ID NO: 1-5, SEQ ID NO: 9 et 10 et SEQ ID NO: 17 et 18 dans le but de détecter la présence d'Enterobactériaceae et de l'association ou non de n'importe lequel des deux genes de virulence *stx* et *eae.*

4. Une méthode suivant la revendication 2, **caractérisée par** l'amplification d'ADN en utilisant une PCR triplex avec les amorces ayant les séquences d'acide nucléique définies suivant SEQ ID NO: 15 et 16, SEQ ID NO: 19 et 20, SEQ ID NO: 23 et 24 et SEQ ID NO: 29 et 30 pour la détection de la presence d'Entérobacteriaceae, *Escherichia coli, Enterococcus faecalis* et *Enterococcus faecium.*

5. Une méthode suivant la revendication 2 ou revendication 3, dans laquelle n'importe laquelle des amorces selon SEQ ID NO: 1 à SEQ ID NO: 5, SEQ ID NO: 31 et SEQ ID NO: 32 sont utilisées pour la détection de tous les variants du gene *stx* par PCR semi-emboîtée duplex.
